(19) Europäisches Patentamt
European Patent Office
Office européen des brevets

(11) EP 3 319 139 A1

(12) EUROPEAN PATENT APPLICATION
published in accordance with Art. 153(4) EPC

(43) Date of publication:
09.05.2018 Bulletin 2018/19

(21) Application number: 16817910.9

(22) Date of filing: 28.06.2016

(51) Int Cl.:
*H01L 51/46* (2006.01) *H01L 51/44* (2006.01)

(86) International application number:
PCT/JP2016/069127

(87) International publication number:
WO 2017/002802 (05.01.2017 Gazette 2017/01)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(30) Priority: 02.07.2015 JP 2015133860

(71) Applicant: Fujifilm Corporation
Minato-ku
Tokyo 106-8620 (JP)

(72) Inventors:
- SATOU, Hirotaka
Ashigarakami-gun
Kanagawa 258-8577 (JP)
- HANAKI, Naoyuki
Ashigarakami-gun
Kanagawa 258-8577 (JP)
- ISE, Toshihiro
Ashigarakami-gun
Kanagawa 258-8577 (JP)

(74) Representative: Hoffmann Eitle
Patent- und Rechtsanwälte PartmbB
Arabellastraße 30
81925 München (DE)

# (54) PHOTOELECTRIC CONVERSION ELEMENT, SOLAR BATTERY, AND COMPOSITION

(57) Provided are a photoelectric conversion element, a solar cell using the photoelectric conversion element, and a composition. The photoelectric conversion element includes a first electrode including a photosensitive layer, which includes a light absorbing agent, on a conductive support. The light absorbing agent includes a compound having a perovskite-type crystal structure that includes organic cations represented by the following Formulae (1) and (2), a cation of a metal atom, and an anion.

Formula (1) $R^1\text{-}N(R^{1a})_3^+$

Formula (2) $R^2\text{-}N(R^{2a})_3^+$

In Formulae (1) and (2), $R^1$ represents a specific group such as an alkyl group (including a specific substituent group in a case where the number of carbons is 1 or 2), and a cycloalkyl group. $R^2$ represents a methyl group, an ethyl group, and the like. $R^{1a}$ and $R^{2a}$ represent a specific group such as a hydrogen atom and an alkyl group.

EP 3 319 139 A1

FIG. 1

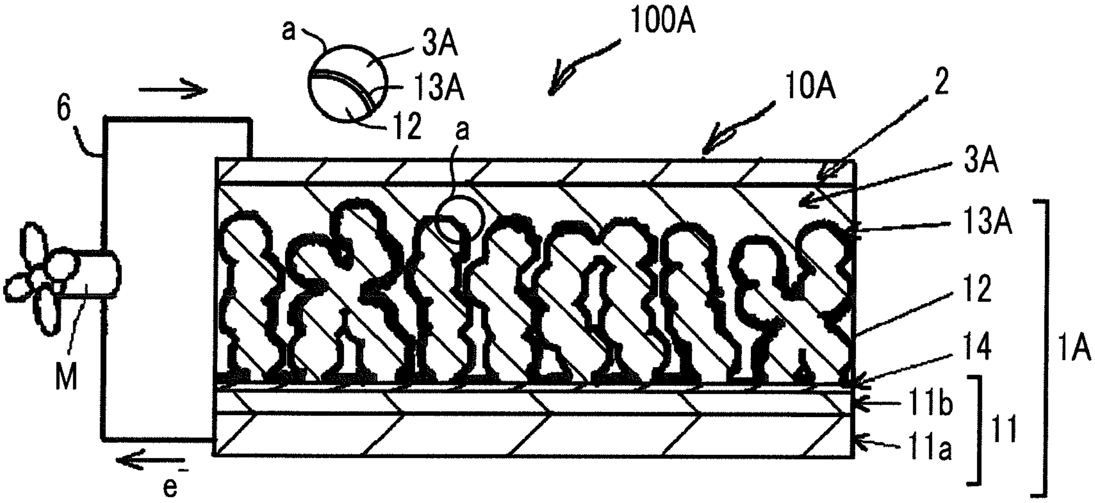

## Description

## BACKGROUND OF THE INVENTION

1. Field of the Invention

The present invention relates to a photoelectric conversion element, a solar cell, and a composition.

2. Description of the Related Art

Photoelectric conversion elements are used in a variety of optical sensors, copiers, solar cells, and the like. It is expected that solar cells will be actively put into practical use as cells using non-exhaustible solar energy. Among these, research and development of dye sensitized solar cells, in which an organic dye, a Ru bipyridyl complex, or the like is used as a sensitizer, are actively in progress, and the photoelectric conversion efficiency thereof reaches approximately 11%.

Meanwhile, in recent years, there have been reported research results indicating that solar cells using a metal halide as a compound having a perovskite-type crystal structure are capable of achieving relatively high photoelectric conversion efficiency, and the solar cells have attracted attention.

For example, a solar cell, which uses a metal halide represented by $CH_3NH_3PbI_2Cl$ as a light absorbing agent, is described in Science, 2012, vol. 338, p. 643 to 647.

In addition, a solar cell, which uses a layered perovskite represented by $(PEA)_2(MA)_2[Pb_3I_{10}]$, is described in Angew. Chem. Int. Ed. 2014, 53, p. 11232 to 11235. Here, PEA represents $C_6H_5(CH_2)_2NH_3^+$, and MA represents $CH_3NH_3^+$.

In addition, a perovskite solar cell, which has a three-layered structure including a $TiO_2$ layer, a $ZrO_2$ layer, and a layer that consists of $(5\text{-AVA})_x(MA)_{1-x}PbI_3$, is described in Science, 2014, vol. 345, p. 295. Here, 5-AVA represents a 5-amino valeryl acid cation, and MA represents a methyl ammonium cation.

## SUMMARY OF THE INVENTION

In a photoelectric conversion element or a solar cell which uses a compound having a perovskite-type crystal structure (hereinafter, referred to as "perovskite compound"), certain results are obtained in an improvement of photoelectric conversion efficiency.

The photoelectric conversion element and the solar cell are required to have durability capable of maintaining initial performance in an in-site environment in which the photoelectric conversion element and the solar cell are actually used. However, it is known that the perovskite compound is easily susceptible to damage in a high-humidity environment. Actually, in the photoelectric conversion element or the solar cell which uses the perovskite compound as a light absorbing agent, there is a concern that photoelectric conversion efficiency greatly deteriorates, particularly, in the high-humidity environment, and a deterioration behavior (deterioration amount) is not constant.

An object of the invention is to provide a photoelectric conversion element which uses a perovskite compound as a light absorbing agent and in which a variation (hereinafter, may be referred to as "moisture resistance variation") in a deterioration amount of photoelectric conversion efficiency is small even under high humidity. In addition, another object of the invention is to provide a solar cell that uses the photoelectric conversion element. In addition, still another object of the invention is to provide a composition that is suitable to form a photosensitive layer of the photoelectric conversion element.

The present inventors found the following finding. With regard to a photoelectric conversion element or a solar cell which uses a perovskite compound having a perovskite-type crystal structure including an organic cation, a metal cation, and an anion as a light absorbing agent, in a case of using two kinds of specific organic cations in combination as the organic cation, a moisture resistance variation is suppressed in the photoelectric conversion element or the solar cell, particularly, even under a high-humidity environment. The invention has been accomplished by additionally repeating examinations on the basis of the finding.

That is, the above-described objects of the invention are accomplished by the following means.

<1> According to an aspect of the invention, there is provided a photoelectric conversion element, comprising: a first electrode that includes a photosensitive layer, which includes a light absorbing agent, on a conductive support; and a second electrode that is opposite to the first electrode.
The light absorbing agent includes a compound having a perovskite-type crystal structure that includes an organic cation, a cation of a metal atom, and an anion of an anionic atom or atomic group.
The organic cation includes an organic cation represented by the following Formula (1) and an organic cation

represented by the following Formula (2).

Formula (1) $R^1\text{-}N(R^{1a})_3^+$

Formula (2) $R^2\text{-}N(R^{2a})_3^+$

In Formulae (1) and (2), $R^1$ represents an alkyl group, a cycloalkyl group, an alkenyl group, an alkynyl group, an aryl group, a heteroaryl group, or an aliphatic heterocyclic group. The alkyl group includes a substituent group, which is selected from the following substituent-group group Z, other than an alkyl group in a case where the number of carbons is 1 or 2.
$R^2$ represents a methyl group, an ethyl group, or a group represented by the following Formula (2a).
$R^{1a}$ and $R^{2a}$ each independently represent a hydrogen atom, an alkyl group, a cycloalkyl group, an alkenyl group, an alkynyl group, an aryl group, a heteroaryl group, or an aliphatic heterocyclic group.

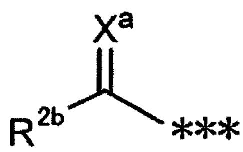

Formula (2a)

In Formula (2a), $X^a$ represents $NR^{2c}$, an oxygen atom, or a sulfur atom. $R^{2b}$ and $R^{2c}$ each independently represent a hydrogen atom or a substituent group. *** represents a bonding portion with a N atom in Formula (2).
Substituent-group group Z: an alkyl group, an alkenyl group, an alkynyl group, an alkoxy group, an aryloxy group, a heteroaryloxy group, an alkylthio group, an arylthio group, a heteroarylthio group, an alkoxycarbonyl group, an aryloxycarbonyl group, a heteroaryloxycarbonyl group, an alkylthiocarbonyl group, an arylthiocarbonyl group, a heteroarylthiocarbonyl group, an alkylcarbonyloxy group, an arylcarbonyloxy group, a heteroarylcarbonyloxy group, an alkylcarbonylthio group, an arylcarbonylthio group, a heteroarylcarbonylthio group, a hydroxy group, a mercapto group, an acyl group, a halogen atom, a cyano group, and a heteroaryl group.
<2> In the photoelectric conversion element according to <1>, in $R^1$, the alkyl group may include a substituent group, which is selected from the substituent-group group Z, other than an alkyl group.
<3> In the photoelectric conversion element according to <1> or <2>, $R^1$ may include at least one selected from the group consisting of an alkoxy group, an alkylthio group, and a halogen atom.
<4> In the photoelectric conversion element according to any one of <1> to <3>, the amount of the organic cation that is contained and is represented by Formula (1), and the amount of the organic cation that is contained and is represented by Formula (2) may satisfy a molar ratio relationship defined by the following expression,

4≤[organic cation represented by Formula (2)]/[organic cation represented by Formula (1)]≤499.

<5> In the photoelectric conversion element according to any one of <1> to <4>, the amount of the organic cation that is contained and is represented by Formula (1), and the amount of the organic cation that is contained and is represented by Formula (2) may satisfy a molar ratio relationship defined by the following expression,

19≤[organic cation represented by Formula (2)]/[organic cation represented by Formula (1)]≤199.

<6> According to another aspect of the invention, there is provided a solar cell that uses the photoelectric conversion element according to any one of <1> to <5>.
<7> According to still another aspect of the invention, there is provided a composition containing:

a compound that is represented by the following Formula (1b); and
a compound that is represented by the following Formula (2b).

Formula (1b) $R^1\text{-}N(R^{1a})_3Hal$

Formula (2b) $R^2\text{-}N(R^{2a})_3Hal$

In Formulae (1b) and (2b), $R^1$ represents an alkyl group, a cycloalkyl group, an alkenyl group, an alkynyl group, an aryl group, a heteroaryl group, or an aliphatic heterocyclic group. The alkyl group includes a substituent group, which is selected from the following substituent-group group Z, other than an alkyl group in a case where the number of carbons is 1 or 2.
$R^2$ represents a methyl group, an ethyl group, or a group represented by the following Formula (2a).
$R^{1a}$ and $R^{2a}$ each independently represent a hydrogen atom, an alkyl group, a cycloalkyl group, an alkenyl group, an alkynyl group, an aryl group, a heteroaryl group, or an aliphatic heterocyclic group.
Hal represents a halogen atom.

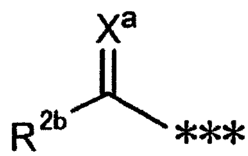
Formula (2a)

In Formula (2a), $X^a$ represents $NR^{2c}$, an oxygen atom, or a sulfur atom, $R^{2b}$ and $R^{2c}$ each independently represent a hydrogen atom or a substituent group. *** represents a bonding portion with a N atom in Formula (2b).
Substituent-group group Z: an alkyl group, an alkenyl group, an alkynyl group, an alkoxy group, an aryloxy group, a heteroaryloxy group, an alkylthio group, an arylthio group, a heteroarylthio group, an alkoxycarbonyl group, an aryloxycarbonyl group, a heteroaryloxycarbonyl group, an alkylthiocarbonyl group, an arylthiocarbonyl group, a heteroarylthiocarbonyl group, an alkylcarbonyloxy group, an arylcarbonyloxy group, a heteroarylcarbonyloxy group, an alkylcarbonylthio group, an arylcarbonylthio group, a heteroarylcarbonylthio group, a hydroxy group, a mercapto group, an acyl group, a halogen atom, a cyano group, and a heteroaryl group.
<8> In the composition according to <7>, in $R^1$, the alkyl group may include a substituent group, which is selected from the substituent-group group Z, other than an alkyl group.
<9> The composition according to <7> or <8> may further contain a halogenated metal.
<10> The composition according to any one of <7> to <9>, the composition may be used to form the photosensitive layer of the photoelectric conversion element according to any one of <1> to <5>.

In this specification, parts of respective formulae may be expressed as a rational formula for understanding of chemical structures of compounds. According to this, in the respective formulae, partial structures are called (substituent) groups, ions, atoms, and the like, but in this specification, the partial structures may represent element groups or elements which constitute (substituent) groups or ions represented by the formulae in addition to the (substituent) groups, the ions, the atoms, and the like.

In this specification, with regard to expression of compounds (including a complex and a dye), the expression is also used to indicate salts of the compounds and ions of the compounds in addition to the compounds. In addition, with regard to compounds for which substitution or non-substitution is not specified, the compounds also include compounds which have an arbitrary substituent group in a range not deteriorating a target effect. This is also true of substituent groups, linking groups, and the like (hereinafter, referred to as "substituent groups and the like").

In this specification, in a case where a plurality of substituent groups and the like expressed using specific symbols or a plurality of substituent groups and the like are simultaneously defined, the respective substituent groups and the like may be identical to or different from each other unless otherwise stated. This is also true of definition of the number of substituent groups and the like. In addition, in a case of approaching each other (particularly, in a case of being close to each other), the plurality of substituent groups and the like may be bonded to each other to form a ring unless otherwise stated. In addition, rings, for example, alicycles, aromatic rings, and hetero rings may be additionally fused together to form a fused ring.

In addition, in this specification, numerical ranges represented by using "to" include ranges including numerical

values before and after "to" as the lower limit and the upper limit.

Even though using a perovskite compound as a light absorbing agent, the photoelectric conversion element and the solar cell of the invention can suppress a moisture resistance variation even under high humidity. In addition, the composition of the invention can be suitably used to form a photosensitive layer in manufacturing of the photoelectric conversion element of the invention.

The above-described and other characteristics and advantages of the invention will be further clarified from the following description with appropriate reference to the accompanying drawings.

## BRIEF DESCRIPTION OF THE DRAWINGS

Fig. 1 is a cross-sectional view schematically illustrating a preferred aspect of a photoelectric conversion element of the invention in addition to an enlarged view of a circle portion in a layer.

Fig. 2 is a cross-sectional view schematically illustrating a preferred aspect of the photoelectric conversion element of the invention in which a thick photosensitive layer is provided.

Fig. 3 is a cross-sectional view schematically illustrating another preferred aspect of the photoelectric conversion element of the invention.

Fig. 4 is a cross-sectional view schematically illustrating still another preferred aspect of the photoelectric conversion element of the invention.

Fig. 5 is a cross-sectional view schematically illustrating still another preferred aspect of the photoelectric conversion element of the invention.

Fig. 6 is a cross-sectional view schematically illustrating still another preferred aspect of the photoelectric conversion element of the invention.

## DESCRIPTION OF THE PREFERRED EMBODIMENTS

«Photoelectric Conversion Element»

A photoelectric conversion element of the invention includes a first electrode including a photosensitive layer, which includes a light absorbing agent, on a conductive support, and a second electrode that is opposite to the first electrode.

In the invention, the aspect in which the photosensitive layer is provided on the conductive support includes an aspect in which the photosensitive layer is provided (directly provided) to be in contact with a surface of the conductive support, and an aspect in which the photosensitive layer is provided on an upper side of the surface of the conductive support through another layer.

In the aspect in which the photosensitive layer is provided on the upper side of the surface of the conductive support through another layer, as the other layer that is provided between the conductive support and the photosensitive layer, there is no particular limitation as long as the other layer does not deteriorate a battery performance of a solar cell. Examples of the other layer include a porous layer, a blocking layer, an electron transport layer, a hole transport layer, and the like.

In the invention, examples of the aspect in which the photosensitive layer is provided on an upper side of the surface of the conductive support through another layer include an aspect in which the photosensitive layer is provided on a surface of a porous layer in a thin film shape (refer to Fig. 1) or in a thick film shape (refer to Fig. 2 and Fig. 6), an aspect in which the photosensitive layer is provided on a surface of a blocking layer in a thin film shape or in a thick film shape (refer to Fig. 3), an aspect in which the photosensitive layer is formed on a surface of an electron transport layer in a thin film shape or a thick film shape (refer to Fig. 4), and an aspect in which the photosensitive layer is provided on a surface of a hole transport layer in a thin film shape or thick film shape (refer to Fig. 5). The photosensitive layer may be provided in a linear shape or in a dispersed pattern, but is preferably provided in a film shape.

In the photoelectric conversion element of the invention, a perovskite compound as a light absorbing agent has a perovskite-type crystal structure including two kinds of specific organic cations, a cation of a metal atom, and an anion of an anionic atom or atomic group. According to this, it is possible to reduce a moisture resistance variation under high humidity in the photoelectric conversion element.

In the photoelectric conversion element of the invention, a configuration other than a configuration defined in the invention is not particularly limited, and it is possible to employ a configuration that is known with respect to the photoelectric conversion element and a solar cell. Respective layers, which constitute the photoelectric conversion element of the invention, are designed in accordance with the purposes thereof, and may be formed, for example, in a monolayer or multilayers. For example, a porous layer may be provided between a conductive support and a photosensitive layer (refer to Fig. 1, Fig. 2, and Fig. 6).

Hereinafter, description will be given of preferred aspects of the photoelectric conversion element of the invention.

In Fig. 1 to Fig. 6, the same reference numeral represents the same constituent element (member).

Furthermore, in Fig. 1, Fig. 2, and Fig. 6, the size of fine particles which form a porous layer 12 is illustrated in a highlighted manner. These fine particles are preferably packed with each other (are deposited or in close contact with each other) in the horizontal direction and the vertical direction with respect to a conductive support 11 to form a porous structure.

In this specification, simple description of "photoelectric conversion element 10" represents photoelectric conversion elements IOA to 10F unless otherwise stated. This is also true of a system 100 and a first electrode 1. In addition, simple description of "photosensitive layer 13" represents photosensitive layers 13A to 13C unless otherwise stated. Similarly, description of "hole transport layer 3" represents hole transport layers 3A and 3B unless otherwise stated.

Examples of a preferred aspect of the photoelectric conversion element of the invention include the photoelectric conversion element 10A illustrated in Fig. 1. A system 100A illustrated in Fig. 1 is a system in which the photoelectric conversion element 10A is applied to a cell that allows operation means M (for example, an electric motor) to operate with an external circuit 6.

The photoelectric conversion element 10A includes a first electrode 1A, a second electrode 2, and the hole transport layer 3A that is provided between the first electrode 1A and the second electrode 2.

The first electrode 1A includes the conductive support 11 including a support 11a and a transparent electrode 11b, the porous layer 12, and a photosensitive layer 13A that is provided with a perovskite-type light absorbing agent on a surface of the porous layer 12 as schematically illustrated in an enlarged cross-sectional region "a" obtained by enlarging the cross-sectional area "a" in Fig. 1. In addition, a blocking layer 14 is provided on the transparent electrode 11b, and the porous layer 12 is formed on the blocking layer 14. As described above, in the photoelectric conversion element 10A including the porous layer 12, it is assumed that a surface area of the photosensitive layer 13A increases, and thus charge separation and charge migration efficiency are improved.

The photoelectric conversion element 10B illustrated in Fig. 2 schematically illustrates a preferred aspect in which the photosensitive layer 13A of the photoelectric conversion element 10A illustrated in Fig. 1 is provided to be thick. In the photoelectric conversion element 10B, the hole transport layer 3B is provided to be thin. The photoelectric conversion element 10B is different from the photoelectric conversion element 10A illustrated in Fig. 1 in the film thickness of the photosensitive layer 13B and the hole transport layer 3B, but the photoelectric conversion element 10B has the same configuration as that of the photoelectric conversion element 10A except for the difference.

The photoelectric conversion element 10C illustrated in Fig. 3 schematically illustrates another preferred aspect of the photoelectric conversion element of the invention. The photoelectric conversion element 10C is different from the photoelectric conversion element 10B illustrated in Fig. 2 in that the porous layer 12 is not provided, but the photoelectric conversion element 10C has the same configuration as that of the photoelectric conversion element 10B except for the difference. That is, in the photoelectric conversion element 10C, the photosensitive layer 13C is formed on the surface of the blocking layer 14 in a thick film shape. In the photoelectric conversion element 10C, the hole transport layer 3B may be provided to be thick in the same manner as in the hole transport layer 3A.

The photoelectric conversion element 10D illustrated in Fig. 4 schematically illustrates still another preferred aspect of the photoelectric conversion element of the invention. The photoelectric conversion element 10D is different from the photoelectric conversion element 10C illustrated in Fig. 3 in that an electron transport layer 15 is provided instead of the blocking layer 14, but the photoelectric conversion element 10D has the same configuration as that of the photoelectric conversion element 10C except for the difference. The first electrode 1D includes the conductive support 11, and the electron transport layer 15 and the photosensitive layer 13C which are sequentially formed on the conductive support 11. The photoelectric conversion element 10D is preferable when considering that the respective layers can be formed from an organic material. According to this, the productivity of the photoelectric conversion element is improved, and thickness reduction or flexibilization becomes possible.

The photoelectric conversion element 10E illustrated in Fig. 5 schematically illustrates still another preferred aspect of the photoelectric conversion element of the invention. A system 100E including the photoelectric conversion element 10E is a system that is applied as a cell in the same manner as in the system 100A.

The photoelectric conversion element 10E includes a first electrode 1E, the second electrode 2, and the electron transport layer 4 that is provided between the first electrode 1E and the second electrode 2. The first electrode 1E includes the conductive support 11, and the hole transport layer 16 and the photosensitive layer 13C which are sequentially formed on the conductive support 11. The photoelectric conversion element 10E is preferable when considering that the respective layers can be formed from an organic material in the same manner as in the photoelectric conversion element 10D.

The photoelectric conversion element 10F illustrated in Fig. 6 schematically illustrates still another preferred aspect of the photoelectric conversion element of the invention. The photoelectric conversion element 10F is different

from the photoelectric conversion element 10B illustrated in Fig. 2 in that the hole transport layer 3B is not provided, but the photoelectric conversion element 10F has the same configuration as that of the photoelectric conversion element 10B except for the difference.

In the invention, a system 100 to which the photoelectric conversion element 10 is applied functions as a solar cell in the following manner.

Specifically, in the photoelectric conversion element 10, light that is transmitted through the conductive support 11, or light that is transmitted through the second electrode 2 and is incident to the photosensitive layer 13 excites a light absorbing agent. The excited light absorbing agent includes high-energy electrons and can emit the electrons. The light absorbing agent, which emits high-energy electrons, becomes an oxidized substance (cation).

In the photoelectric conversion elements 10A to 10D, and 10F, electrons emitted from the light absorbing agent migrate between a plurality of the light absorbing agents and reach the conductive support 11. The electrons which have reached the conductive support 11 work in the external circuit 6, and then return to the photosensitive layer 13 through the second electrode 2 (in a case where the hole transport layer 3 is provided, additionally through the hole transport layer 3). The light absorbing agent is reduced by the electrons which have returned to the photosensitive layer 13.

On the other hand, in the photoelectric conversion element 10E, the electrons, which are emitted from the light absorbing agent, reach the second electrode 2 from the photosensitive layer 13C through the electron transport layer 4, and work in the external circuit 6. Then, the electrons return to the photosensitive layer 13 through the conductive support 11. The light absorbing agent is reduced by the electrons which have returned to the photosensitive layer 13.

As described above, in the photoelectric conversion element 10, a cycle of excitation of the light absorbing agent and electron migration is repeated, and thus the system 100 functions as a solar cell.

In the photoelectric conversion elements 10A to 10D, and 10F, a method of allowing an electron to flow from the photosensitive layer 13 to the conductive support 11 is different depending on presence or absence of the porous layer 12, a kind thereof, and the like. In the photoelectric conversion element 10 of the invention, electron conduction, in which electrons migrate between the light absorbing agents, occurs. In addition, in the invention, in a case where the porous layer 12 is provided, the porous layer 12 can be formed from an insulating substance other than semiconductors in the related art. In a case where the porous layer 12 is formed from a semiconductor, electron conduction, in which electrons migrate at the inside of semiconductor fine particles of the porous layer 12 or between the semiconductor fine particles, also occurs. On the other hand, in a case where the porous layer 12 is formed from an insulating substance, electron conduction in the porous layer 12 does not occur. In a case where the porous layer 12 is formed from the insulating substance, in a case of using fine particles of an aluminum oxide ($Al_2O_3$) as the fine particles of the insulating substance, a relatively high electromotive force ($V_{OC}$) is obtained.

Even in a case where the blocking layer 14 as the other layer is formed from a conductor or a semiconductor, electron conduction in the blocking layer 14 occurs.

In addition, even in the electron transport layer 15, electron conductor occurs.

The photoelectric conversion element and the solar cell of the invention are not limited to the preferred aspects, and configurations and the like of the respective aspects may be appropriately combined between the respective aspects in a range not departing from the gist of the invention. For example, the photoelectric conversion element 10C or 10D may have a configuration in which the hole transport layer 3B is not provided in the same manner as in the photoelectric conversion element 10F.

In the invention, materials and respective members which are used in the photoelectric conversion element and the solar cell can be prepared by using a typical method except for the light absorbing agent. With regard to a photoelectric conversion element or a solar cell in which a perovskite compound is used, for example, reference can be made to Science, 2012, vol. 338, p. 643 to 647, Angew. Chem. Int. Ed. 2014, 53, p. 11232 to 11235, Science, 2014, vol. 345, p. 295, J. Am. Chem. Soc., 2009, 131(17), p. 6050 to 6051, and Science, 338, p. 643(2012).

In addition, reference can be made to materials and respective members which are used in a dye sensitized solar cell. With regard to dye sensitized solar cells, for example, reference can be made to JP2001-291534A, US4,927,721A, US4,684,537A, US5,084,365A, US5,350,644A, US5,463,057A, US5,525,440A, JP1995-249790A (JP-H7-249790A), JP2004-220974A, and JP2008-135197A.

Hereinafter, description will be given of preferred aspects of members and compounds which are appropriately used in the photoelectric conversion element and the solar cell of the invention.

<First Electrode 1>

The first electrode 1 includes the conductive support 11 and the photosensitive layer 13, and functions as a working electrode in the photoelectric conversion element 10.

As illustrated in Fig. 1 to Fig. 6, it is preferable that the first electrode I includes at least one of the porous layer 12, the blocking layer 14, the electron transport layer 15, or the hole transport layer 16.

It is preferable that the first electrode 1 includes at least the blocking layer 14 from the viewpoint of short-circuit

prevention, and more preferably the porous layer 12 and the blocking layer 14 from the viewpoints of light absorption efficiency and short-circuit prevention.

In addition, it is preferable that the first electrode 1 includes the electron transport layer 15 or the hole transport layer 16, which is formed from an organic material, from the viewpoints of an improvement in productivity of the photoelectric conversion element, thickness reduction, and flexibilization.

-Conductive Support 11 -

The conductive support 11 is not particularly limited as long as the conductive support 11 has conductivity and can support the photosensitive layer 13 and the like. It is preferable that the conductive support 11 has a configuration formed from a conductive material, for example, a metal, or a configuration including the support 11a formed from glass or plastic and the transparent electrode 11b formed on a surface of the support 11a as a conductive film. In a case where the strength of the conductive support 11 is sufficiently maintained, the support 11a is not necessary.

Among these, as illustrated in Fig. 1 to Fig. 6, it is more preferable that the conductive support 11 has a configuration in which a conductive metal oxide is applied to the surface of the support 11a formed from glass or plastic to form the transparent electrode 11b. Examples of the support 11a formed from plastic include a transparent polymer film described in Paragraph 0153 of JP2001-291534A. As a material that forms the support 11a, it is possible to use ceramic (JP2005-135902A) and a conductive resin (JP2001-160425A) in addition to glass or plastic. As a metal oxide, a tin oxide (TO) is preferable, and an indium-tin oxide (a tin-doped indium oxide; ITO) or a tin oxide doped with fluorine such as a fluorine-doped tin oxide (FTO) is more preferable. At this time, the amount of the metal oxide applied is preferably 0.1 to 100 g per square meter of a surface area of the support 11a. In a case of using the conductive support 11, it is preferable that light is incident from a support 11a side.

It is preferable that the conductive support 11 is substantially transparent. In the invention, "substantially transparent" represents that transmittance of light (having a wavelength of 300 to 1200 nm) is 10% or greater, preferably 50% or greater, and more preferably 80% or greater.

The thickness of the support 11a and the conductive support 11 is not particularly limited and is set to an appropriate thickness. For example, the thickness is preferably 0.01 μm to 10 mm, more preferably 0.1 μm to 5 mm, and still preferably 0.3 μm to 4 mm.

In a case of providing the transparent electrode 11b, the film thickness of the transparent electrode 11b is not particularly limited. For example, the film thickness is preferably 0.01 to 30 μm, more preferably 0.03 to 25 μm, and still more preferably 0.05 to 20 μm.

The conductive support 11 or the support 11a may have a light management function on the surface. For example, the conductive support 11 or the support 11a may include an antireflection film formed by alternately laminating a high-refractive-index film and a low-refractive-index oxide film on the surface of the conductive support 11 or the support 11a as described in JP2003-123859A or may have a light guide function as described in JP2002-260746A.

- Blocking Layer 14 -

In the invention, as in the photoelectric conversion elements 10A to 10C, and 10F, the blocking layer 14 is preferably provided on the surface of the transparent electrode 11b, that is, between the conductive support 11, and the porous layer 12, the photosensitive layer 13, the hole transport layer 3, or the like.

In the photoelectric conversion element and the solar cell, for example, in a case where the photosensitive layer 13 or the hole transport layer 3, and the transparent electrode 11b and the like are electrically connected to each other, a reverse current is generated. The blocking layer 14 plays a role of preventing the reverse current. The blocking layer 14 is also referred to as a "short-circuit prevention layer".

The blocking layer 14 may be allowed to function as a stage that carries the light absorbing agent.

The blocking layer 14 may be provided even in a case where the photoelectric conversion element includes the electron transport layer. For example, in a case of the photoelectric conversion element 10D, the blocking layer 14 may be provided between the conductive support 11 and the electron transport layer 15. In a case of the photoelectric conversion element 10E, the blocking layer 14 may be provided between the second electrode 2 and the electron transport layer 4.

The material that forms the blocking layer 14 is not particularly limited as long as the material can perform the above-described function, and it is preferable that the material is a material through which visible light is transmitted, and which has insulating properties with respect to the conductive support 11 (transparent electrode 11b) and the like. Specifically, "material having insulating properties with respect to the conductive support 11 (transparent electrode 11b)" represents a compound (n-type semiconductor compound) having a conduction band energy level that is equal to or higher than a conduction band energy level of a material that forms the conductive support 11 (a metal oxide that forms the transparent electrode 11b) and the like and is lower than a conduction band of a material that constitutes the porous

layer 12 and the like or a ground state energy level of the light absorbing agent.

Examples of a material that forms the blocking layer 14 include silicon oxide, magnesium oxide, aluminum oxide, calcium carbonate, cesium carbonate, polyvinyl alcohol, polyurethane, and the like. In addition, the material may be a material that is typically used as a photoelectric conversion material, and examples thereof include titanium oxide, tin oxide, zinc oxide, niobium oxide, tungsten oxide, and the like. Among these, titanium oxide, tin oxide, magnesium oxide, aluminum oxide, and the like are preferred.

It is preferable that the film thickness of the blocking layer 14 is 0.001 to 10 μm, more preferably 0.005 to 1 μm, and still more preferably 0.01 to 0.1 μm.

In the invention, the film thicknesses of the respective layers can be measured by observing a cross-section of the photoelectric conversion element 10 by using a scanning electron microscope (SEM) and the like.

- Porous Layer 12 -

In the invention, as in the photoelectric conversion elements 10A, 10B, and 10F, the porous layer 12 is preferably provided on the transparent electrode 11b. In a case where the blocking layer 14 is provided, the porous layer 12 is preferably formed on the blocking layer 14.

The porous layer 12 is a layer that functions as a stage that carries the photosensitive layer 13 on the surface. In a solar cell, so as to increase the light absorption efficiency, it is preferable to increase a surface area of at least a portion that receives light such as solar light, and it is more preferable to increase the surface area of the porous layer 12 as a whole.

It is preferable that the porous layer 12 is a fine particle layer that includes pores and is formed through vapor deposition or close contact of fine particles of a material that forms the porous layer 12. The porous layer 12 may be a fine particle layer that is formed through vapor deposition of two or more kinds of fine particles. In a case where the porous layer 12 is a fine particle layer that includes pores, it is possible to increase the amount (adsorption amount) of the carried light absorbing agent.

It is preferable to increase the surface area of individual fine particles which constitute the porous layer 12 so as to increase the surface area of the porous layer 12. In the invention, in a state in which the fine particles are applied to the conductive support 11 and the like, it is preferable that the surface area of the fine particles which form the porous layer 12 is 10 or more times a projected area, and more preferably 100 or more times the projected area. The upper limit thereof is not particularly limited. Typically, the upper limit is approximately 5000 times the projected area. With regard to a particle size of the fine particles which form the porous layer 12, an average particle size, which uses a diameter when converting the projected area into a circle, is preferably 0.001 to 1 μm as primary particles. In a case where the porous layer 12 is formed by using a dispersion of fine particles, the average particle size of the fine particles is preferably 0.01 to 100 μm in terms of an average particle size of the dispersion.

For the material that forms the porous layer 12, there is no particular limitation with respect to conductivity. The material may be an insulating substance (insulating material), a conductive material, or a semiconductor (semi-conductive material).

As the material that forms the porous layer 12, it is possible to use, for example, chalcogenides (for example, an oxide, a sulfide, a selenide, and the like) of metals, compounds having a perovskite-type crystal structure (excluding a perovskite compound that uses a light absorbing agent), oxides of silicon (for example, silicon dioxide, and zeolite), or carbon nanotubes (including carbon nanowires, carbon nanorods, and the like).

The chalcogenides of a metal are not particularly limited, and preferred examples thereof include respective oxides of titanium, tin, zinc, tungsten, zirconium, hafnium, strontium, indium, cerium, yttrium, lanthanum, vanadium, niobium, aluminum, and tantalum, cadmium sulfide, cadmium selenide, and the like. Examples of the crystal structure of the chalcogenides of metals include an anatase-type crystal structure, a brookite-type crystal structure, and a rutile-type crystal structure, and the anatase-type crystal structure and the brookite-type crystal structure are preferable.

The compound having a perovskite-type crystal structure is not particularly limited, and examples thereof include a transition metal oxide and the like. Examples of the transition metal oxide include strontium titanate, calcium titanate, barium titanate, lead titanate, barium zirconate, barium stannate, lead zirconate, strontium zirconate, strontium tantalate, potassium niobate, bismuth ferrate, barium strontium titanate, lanthanum barium titanate, calcium titanate, sodium titanate, and bismuth titanate. Among these, strontium titanate, calcium titanate, and the like are preferable.

The carbon nanotubes have a shape obtained by rounding off a carbon film (graphene sheet) into a tubular shape. The carbon nanotubes are classified into a single-walled carbon nanotube (SWCNT) obtained by winding one graphene sheet in a cylindrical shape, a double-walled carbon nanotube (DWCNT) obtained by winding two graphene sheets in a concentric shape, and a multi-walled carbon nanotube (MWCNT) obtained by winding a plurality of graphene sheets in a concentric shape. As the porous layer 12, any carbon nanotubes can be used without any particular limitation.

Among these, as the material that forms the porous layer 12, an oxide of titanium, tin, zinc, zirconium, aluminum, or silicon, or a carbon nanotube is preferable, and titanium oxide or aluminum oxide is more preferable.

The porous layer 12 may be formed from at least one kind of the chalcogenides of metals, the compound having a perovskite-type crystal structure, the oxide of silicon, or the carbon nanotube, or may be formed from a plurality of kinds thereof.

The film thickness of the porous layer 12 is not particularly limited. The film thickness is typically in a range of 0.05 to 100 μm, and preferably in a range of 0.1 to 100 μm. In a case of being used as a solar cell, the film thickness is preferably 0.1 to 50 μm, and more preferably 0.2 to 30 μm.

- Electron Transport Layer 15 -

In the invention, as in the photoelectric conversion element 10D, the electron transport layer 15 is preferably provided on the surface of the transparent electrode 11b.

The electron transport layer 15 has a function of transporting electrons, which are generated in the photosensitive layer 13, to the conductive support 11. The electron transport layer 15 is formed from an electron transporting material capable of exhibiting the above-described function. The electron transporting material is not particularly limited, and an organic material (organic electron transporting material) is preferable. Examples of the organic electron transporting material include fullerene compounds such as [6,6]-phenyl-C61-butyric acid methyl ester ($PC_{61}BM$), perylene compounds such as perylene tetracarboxylic diimide (PTCDI), low-molecular-weight compounds such as tetracyanoquinodimethane (TCNQ), high-molecular-weight compounds, and the like.

Although not particularly limited, it is preferable that the film thickness of the electron transport layer 15 is 0.001 to 10 μm, and more preferably 0.01 to 1 μm.

- Hole Transport Layer 16 -

In the invention, as in the photoelectric conversion element 10E, the hole transport layer 16 is preferably provided on the surface of the transparent electrode 11b.

The hole transport layer 16 is the same as the hole transport layer 3 to be described later except for a different formation position.

- Photosensitive Layer (Light Absorbing Layer) 13 -

The photosensitive layer 13 is preferably provided on the surface (including an inner surface of a concave portion in a case where a surface on which the photosensitive layer 13 is provided is uneven) of each of the porous layer 12 (in the photoelectric conversion elements 10A, 10B, and 10F), the blocking layer 14 (in the photoelectric conversion element 10C), the electron transport layer 15 (in the photoelectric conversion element 10D), and the hole transport layer 16 (in the photoelectric conversion element 10E).

In the invention, the light absorbing agent may contain a perovskite compound that includes at least one kind of organic cation (hereinafter, may be referred to as "an organic cation (A1)) represented by the following Formula (1), and at least one kind of organic cation (hereinafter, may be referred to as "organic cation (A2)) represented by the following Formula (2).

In addition, the light absorbing agent may include a light absorbing agent other than the perovskite compound in combination with the perovskite compound. Examples of the light absorbing agent other than the perovskite compound include a metal complex dye, and an organic dye. At this time, a ratio between the perovskite compound and the light absorbing agent other than the perovskite compound is not particularly limited.

The photosensitive layer 13 may be a monolayer or a laminated layer of two or more layers. In a case where the photosensitive layer 13 has the laminated layer structure of two or more layers, the laminated layer structure may be a laminated layer structure obtained by laminating layers which consist of light absorbing agents different from each other, or a laminated layer structure including an interlayer including a hole transporting material between a photosensitive layer and a photosensitive layer. In a case where the photosensitive layer 13 has the laminated structure of two or more layers, the photosensitive layer 13 may include the perovskite compound including at least one kind of the organic cation (A1) and at least one kind of the organic cation (A2) in any layer as long as the perovskite compound is included in at least one layer, or in all layers.

The aspect in which the photosensitive layer 13 is provided on the conductive support 11 is as described above. The photosensitive layer 13 is preferably provided on a surface of each of the layers in order for an excited electron to flow to the conductive support 11 or the second electrode 2. At this time, the photosensitive layer 13 may be provided on the entirety or a part of the surface of each of the layers.

The film thickness of the photosensitive layer 13 is appropriately set in correspondence with an aspect in which the photosensitive layer 13 is provided on the conductive support 11, and is not particularly limited. Typically, for example, the film thickness is preferably 0.001 to 100 μm, more preferably 0.01 to 10 μm, and still more preferably 0.01 to 5 μm.

In a case where the porous layer 12 is provided, a total film thickness including the film thickness of the porous layer 12 is preferably 0.01 μm or greater, more preferably 0.05 μm or greater, still more preferably 0.1 μm or greater, and still more preferably 0.3 μm or greater. In addition, the total film thickness is preferably 100 μm or less, more preferably 50 μm or less, and still more preferably 30 μm or less. The total film thickness may be set to a range in which the above-described values are appropriately combined. Here, as illustrated in Fig. 1, in a case where the photosensitive layer 13 has a thin film shape, the film thickness of the photosensitive layer 13 represents a distance between an interface with the porous layer 12, and an interface with the hole transport layer 3 to be described later along a direction that is perpendicular to the surface of the porous layer 12.

In the photoelectric conversion element 10, in a case where the porous layer 12 and the hole transport layer 3 are provided, a total film thickness of the porous layer 12, the photosensitive layer 13, and the hole transport layer 3 is not particularly limited. For example, the total thickness is preferably 0.01 μm or greater, more preferably 0.05 μm or greater, still more preferably 0.1 μm or greater, and still more preferably 0.3 μm or greater. In addition, the total film thickness is preferably 200 μm or less, more preferably 50 μm or less, still more preferably 30 μm or less, and still more preferably 5 μm or less. The total film thickness can be set to a range in which the above-described values are appropriately combined.

In the invention, in a case where the photosensitive layer is provided in a thick film shape (in the photosensitive layer 13B and 13C), the light absorbing agent that is included in the photosensitive layer may function as a hole transporting material.

[Light Absorbing Agent]

The photosensitive layer 13 contains a perovskite compound (also referred to as "perovskite-type light absorbing agent) that includes "a cationic organic group A", "a metal atom M", and "an anionic atom or atomic group X" as the light absorbing agent.

In a perovskite-type crystal structure, the cationic organic group A, the metal atom M, and the anionic atom or atomic group X of the perovskite compound exist as individual constituent ions of an organic cation (for convenience, may be referred to as "organic cation A"), a metal cation (for convenience, may be referred to as "metal cation M"), and an anion (for convenience, may be referred to as "anion X").

In the invention, the cationic organic group represents an organic group having a property of becoming the organic cation A in the perovskite-type crystal structure. The cationic organic group A includes at least two kinds of organic groups which respectively generate at least two kinds of organic cations to be described later.

The anionic atom or atomic group represents an atom or atomic group that has a property of becoming an anion in the perovskite-type crystal structure.

In the photoelectric conversion element of the invention, the perovskite compound as the light absorbing agent has a perovskite-type crystal structure including two kinds of specific organic cations (A1) and (A2), a cation of a metal atom, and an anion of an anionic atom or atomic group. According to this, it is possible to reduce a moisture resistance variation under high humidity in the photoelectric conversion element and the like.

In a photoelectric conversion element or a solar cell that uses a perovskite compound of the related art, the perovskite compound is easily decomposed due to moisture that exists at the outside or moisture that exists at the inside in advance. Particularly, photoelectric conversion efficiency greatly deteriorates under a high-humidity environment. The reason for this is considered to be because a defect occurs in an interface between the photosensitive layer and a layer (for example, the hole transport layer 3, the electron transport layer 4, or the second electrode 2) adjacent to the photosensitive layer, and decomposition of the perovskite compound rapidly progresses due to intrusion of water into the photosensitive layer from the interface defect, and the like.

However, in a case of using two kinds of organic cation (A1) and (A2), which include a specific group ($R^1$ or $R^2$) and are different in hydrophilicity or hydrophobicity and in a three-dimensional bulk, in combination, an organic cation that is suitable for a surface state of an adjacent layer can come into contact with the adjacent layer, and thus an effect of improving affinity between the photosensitive layer and the adjacent layer is exhibited. It is considered that it is possible to suppress generation of the interface defect with reproducibility due to the affinity improving effect. According to this, in the photoelectric conversion element and the solar cell of the invention, the moisture resistance variation is reduced even under a high-humidity environment, and thus a moisture resistant performance becomes excellent.

In the invention, the high-humidity environment (high humidity) is not particularly limited, and represents an environment (humidity) of 50%RH or greater as an example.

The organic cation (A1) is represented by the following Formula (1).

Formula (1) $R^1\text{-}N(R^{1a})_3^+$

In Formula (1), $R^1$ represents an alkyl group, a cycloalkyl group, an alkenyl group, an alkynyl group, an aryl

group, a heteroaryl group, or an aliphatic heterocyclic group. The alkyl group includes a substituent group, which is selected from the following substituent-group group Z, other than an alkyl group in a case where the number of carbons is 1 or 2 (methyl or ethyl).

The alkyl group that can be employed as $R^1$ includes a linear alkyl group and a branched alkyl group. The number of carbons of the alkyl group is preferably 1 to 30, more preferably 1 to 18, still more preferably 1 to 12, and still more preferably 2 to 6. Specific preferred examples of the alkyl group include methyl, ethyl, propyl, isopropyl, n-butyl, t-butyl, pentyl, hexyl, decyl, and octadecyl.

Here, methyl and ethyl include a substituent group, which is selected from the following substituent-group group Z, other than an alkyl group. The substituent group is directly bonded to methyl or ethyl. That is, in a case where the entirety of $R^1$ is composed of an alkyl group, the entirety of $R^1$ is interpreted as one alkyl group, and it is not interpreted that methyl or ethyl includes a remaining alkyl group as a substituent group. In the substituent group included in each of methyl and ethyl, preferred groups and preferred ranges of respective groups are the same as preferred groups and preferred ranges which are described in the following substituent-group group Z. As to be described later, methyl and ethyl may include a substituent group (including an alkyl group) selected from the substituent-group group Z as long as the substituent group is a substituent group (a composite substituent group to be described later) that is bonded to methyl or ethyl through another substituent group.

It is preferable that the alkyl group (including an alkyl group having 3 or more carbon atoms) that can be employed as $R^1$ includes a substituent group, which is selected from the following substituent-group group Z, other than an alkyl group. Even in this case, the substituent group is directly bonded to the alkyl group. In the substituent group that is preferably included in the alkyl group, preferred groups and preferred ranges of respective groups are the same as preferred groups and preferred ranges which are described in the following substituent-group group Z. As to be described later, the alkyl group may include a substituent group (including an alkyl group) selected from the substituent-group group Z as long as the substituent group is a substituent group (a composite substituent group to be described later) that is bonded to the alkyl group through another substituent group.

The cycloalkyl group that can be employed as $R^1$ is preferably a cycloalkyl group having 3 to 8 carbon atoms. Specific preferred examples of the cycloalkyl group include cyclopropyl, cyclopentyl, and cyclohexyl.

The alkenyl group that can be employed as $R^1$ includes a linear alkenyl group and a branched alkenyl group. The number of carbons of the alkenyl group is preferably 2 to 18, more preferably 2 to 7, and still more preferably 2 to 5. Specific preferred examples of the alkenyl group include vinyl, allyl, butenyl, and hexenyl.

The alkynyl group that can be employed as $R^1$ includes a linear alkynyl group and a branched alkynyl group. The number of carbons of the alkynyl group is preferably 2 to 18, more preferably 2 to 7, and still more preferably 2 to 5. Specific preferred examples of the alkynyl group include ethynyl, propynyl, butynyl, hexynyl, and octynyl.

The aryl group that can be employed as $R^1$ is preferably an aryl group having 6 to 14 carbon atoms. Specific preferred examples of the aryl group include phenyl and naphthyl, and phenyl is more preferable.

The heteroaryl group that can be employed as $R^1$ includes a group that consists of an aromatic hetero ring alone, and a group that consists of a condensed hetero ring obtained through condensing of another ring, for example, an aromatic ring, an aliphatic ring, or a hetero ring with the aromatic hetero ring.

As the ring-constituting hetero atom that constitutes the aromatic hetero ring, a nitrogen atom, an oxygen atom, or a sulfur atom is preferable. In addition, with regard to the number of ring members of the aromatic hetero ring, three-membered to eight-membered rings are preferable, and a five-membered ring or a six-membered ring is more preferable.

Examples of the five-membered aromatic hetero ring and the condensed hetero ring including the five-membered aromatic hetero ring include respective cyclic groups of a pyrrole ring, an imidazole ring, a pyrazole ring, an oxazole ring, a thiazole ring, a triazole ring, a furan ring, a thiophene ring, a benzimidazole ring, a benzoxazole ring, a benzothiazole ring, an indoline ring, and an indazole ring. In addition, examples of the six-membered aromatic hetero ring and the condensed hetero ring including the six-membered aromatic hetero ring include respective cyclic groups of a pyridine ring, a pyrimidine ring, a pyrazine ring, a triazine ring, a quinoline ring, and a quinazoline ring.

The aliphatic heterocyclic group that can be employed as $R^1$ includes a group that consists of an aliphatic hetero ring alone, and a group that consists of a condensed aliphatic hetero ring obtained through condensing of another ring, for example, an aliphatic ring with the aliphatic hetero ring. As the ring-constituting hetero atom that constitutes the aliphatic hetero ring, a nitrogen atom, an oxygen atom, or a sulfur atom is preferable. In addition, with regard to the number of ring members of the aliphatic hetero ring, three-membered to eight-membered rings are preferable, and a five-membered ring or a six-membered ring is more preferable. The number of carbons of the aliphatic hetero ring is preferably 0 to 24, and more preferably 1 to 18.

Specific preferred examples of the aliphatic hetero ring include a pyrrolidine ring, an oxolane ring, a thiolane ring, a piperidine ring, a tetrahydrofuran ring, an oxane ring, a dioxane ring, a thiane ring, a piperazine ring, a morpholine ring, a quinuclidine ring, an azetidine ring, an oxetane ring, an aziridine ring, a pentamethyl sulfide ring, $\gamma$-butyrolactone, and the like.

Among these, as $R^1$, an alkyl group, a cycloalkyl group, an alkenyl group, an aryl group, a heteroaryl group, or

an aliphatic heterocyclic group is preferable, the alkyl group is more preferable, and methyl, ethyl, or propyl is still more preferable.

$R^1$ (the respective groups which can be employed as $R^1$) may include a substituent group. The substituent group in this case is not particularly limited, but a substituent group selected from the following substituent-group group Z is preferable.

An aspect in which $R^1$ includes a substituent group is not particularly limited, and examples thereof include an aspect in which $R^1$ includes a substituent group (an independent substituent group that is not bonded to another substituent group), an aspect in which $R^1$ includes a composite substituent group obtained in combination of two or more substituent groups, and an aspect in which $R^1$ includes a substituent group (an independent substituent group) and a composite substituent group.

In the invention, in a case where $R^1$ includes a substituent group, $R^1$ and the substituent group are determined so that the number of carbons increases in the order of $R^1$ and a substituent group that is directly bonded to $R^1$ as necessary. Even in the case of the composite substituent group, respective substituent groups are determined in the same manner as necessary so that the number of carbons further increases in a substituent group that is closer to $R^1$.

The substituent-group group Z includes an alkyl group, an alkenyl group, an alkynyl group, an alkoxy group, an aryloxy group, a heteroaryloxy group, an alkylthio group, an arylthio group, a heteroarylthio group, an alkoxycarbonyl group, an aryloxycarbonyl group, a heteroaryloxycarbonyl group, an alkylthiocarbonyl group, an arylthiocarbonyl group, a heteroarylthiocarbonyl group, an alkylcarbonyloxy group, an arylcarbonyloxy group, a heteroarylcarbonyloxy group, an alkylcarbonylthio group, an arylcarbonylthio group, a heteroarylcarbonylthio group, a hydroxy group, a mercapto group, an acyl group, a halogen atom, a cyano group, and a heteroaryl group.

In the invention, in a case where the alkyl group is described in distinction from the cycloalkyl group, the alkyl group is used to include a linear alkyl group and a branched alkyl group. On the other hand, in a case where the alkyl group is not described in distinction from the cycloalkyl group (in a case of description simply as an alkyl group), the alkyl group is used to include a linear alkyl group, a branched alkyl group, and a cycloalkyl group. This is also true of a group (an alkoxy group, an alkylthio group, an alkenyloxy group, or the like) including a group (an alkyl group, an alkenyl group, an alkynyl group, or the like) that can employ a cyclic structure, and a compound including a group that can employ a cyclic structure. In description of the following substituent-group group Z, the alkyl group is described to include a cycloalkyl group without any particular notation.

In the substituent-group group Z, preferred ranges of the alkyl group (including the cycloalkyl group), the alkenyl group, the alkynyl group, and the heteroaryl group are the same as the preferred ranges of the corresponding respective groups in $R^1$. However, even in a case where the alkyl group is composed of methyl or ethyl, a substituent group is not essential differently from the alkyl group of $R^1$.

The number of carbons of the acyl group is preferably 1 to 19, and more preferably 2 to 19. Specific preferred examples of the acyl group include formyl, acetyl, propionyl, octadecanoyl, benzoyl, and acryloyl.

Examples of the halogen atom include a fluorine atom, a chlorine atom, a bromine atom, and an iodine atom, and the iodine atom or the bromine atom is preferable.

In the invention, in the substituent-group group Z, an alkoxy group, an aryloxy group, a heteroaryloxy group, an alkylthio group, an arylthio group, a heteroarylthio group, an alkoxycarbonyl group, an aryloxycarbonyl group, a heteroaryloxycarbonyl group, an alkylthiocarbonyl group, an arylthiocarbonyl group, a heteroarylthio carbonyl group, an alkylcarbonyloxy group, an arylcarbonyloxy group, a heteroarylcarbonyloxy group, an alkylcarbonylthio group, an arylcarbonylthio group, and a heteroarylcarbonylthio group can be individually represented by any one of the following Formulae (W-1) to (W-6).

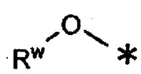
(W-1)

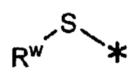
(W-2)

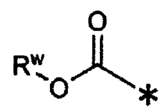
(W-3)

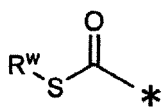
(W-4)

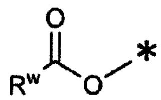
(W-5)

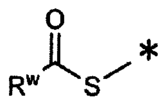
(W-6)

In Formulae (W-1) to (W-6), * represents a N atom in Formula (1), or a linking portion with another substituent group that is selected from the substituent-group group Z.

$R^w$ represents any one of an alkyl group, an aryl group, and a heteroaryl group. Preferred ranges of the alkyl

group, the aryl group, and the heteroaryl group are the same as the preferred ranges of the alkyl group (including the cycloalkyl group), the aryl group, and the heteroaryl group of $R^1$. However, even in a case where the alkyl group is composed of methyl or ethyl, a substituent group is not essential differently from the alkyl group of $R^1$.

A correlation between the respective substituent groups included in the substituent-group group Z, and the groups represented by Formulae (W-1) to (W-6) is illustrated in the following Table 1.

[Table 1]

| Substituent group included in substituent-group group Z | Formula | Rw |
|---|---|---|
| Alkoxy group | W-1 | Alkyl |
| Aryloxy group | W-1 | Aryl |
| Heteroaryloxy group | W-1 | Heteroaryl |
| Alkylthio group | W-2 | Alkyl |
| Arylthio group | W-2 | Aryl |
| Heteroarylthio group | W-2 | Heteroaryl |
| Alkoxycarbonyl group | W-3 | Alkyl |
| Aryloxycarbonyl group | W-3 | Aryl |
| Heteroaryloxycarbonyl group | W-3 | Heteroaryl |
| Alkylthiocarbonyl group | W-4 | Alkyl |
| Arylthiocarbonyl group | W-4 | Aryl |
| Heteroarylthiocarbonyl group | W-4 | Heteroaryl |
| Alkylcarbonyloxy group | W-5 | Alkyl |
| Arylcarbonyloxy group | W-5 | Aryl |
| Heteroarylcarbonyloxy group | W-5 | Heteroaryl |
| Alkylcarbonylthio group | W-6 | Alkyl |
| Arylcarbonylthio group | W-6 | Aryl |
| Heteroarylcarbonylthio group | W-6 | Heteroaryl |

Even in any of the aspects, as a substituent group selected from the substituent-group group Z, an alkoxy group, an alkylthio group, or a halogen atom is preferable.

The substituent group selected from the substituent-group group Z may further include a substituent group (in an aspect including a composite substituent group). In the aspect, the substituent group that can be further included is not particularly limited, and preferred examples thereof include a substituent group selected from the substituent-group group Z. A preferred range of the substituent group that can be further included is the same as the preferred range of the substituent-group group Z.

In the above-described aspects, a combination of substituent groups which are selected from a plurality of substituent-group groups Z is not particularly limited, and examples thereof include a combination of a halogen atom and another substituent group, a combination of an alkyl group and another substituent group, or a combination of an alkylthio group and another substituent group in addition to combinations in specific examples to be described later.

Examples of the combination of the halogen atom and another substituent group include a combination of an alkyl group and a halogen atom, a combination of an alkoxy group and a halogen atom, a combination of an aryloxy group and a halogen atom, a combination of a heteroaryloxy group and a halogen atom, a combination of an alkylthio group and a halogen atom, a combination of an arylthio group and a halogen atom, a combination of a heteroarylthio group and a halogen atom, a combination of an acyl group and a halogen atom, and a combination of a heteroaryl group and a halogen atom.

Examples of the combination of the alkyl group and another substituent group include a combination of an aryloxy group and an alkyl group, a combination of a heteroaryloxy group and an alkyl group, a combination of an arylthio group and an alkyl group, a combination of a heteroarylthio group and an alkyl group, a combination of an aryloxycarbonyl group and an alkyl group, a combination of a heteroaryloxycarbonyl group and an alkyl group, a combination of an arylthiocarbonyl group and an alkyl group, a combination of a heteroarylthiocarbonyl group and an alkyl group, a combination of an arylcarbonyloxy group and an alkyl group, a combination of a heteroarylcarbonyloxy group and an alkyl group, a combination of an arylcarbonylthio group and an alkyl group, a combination of a heteroarylcarbonylthio group and an alkyl group, and a combination of a heteroaryl group and an alkyl group.

Examples of the combination of the alkylthio group and another substituent group include a combination of an aryloxy group and an alkylthio group, a combination of a heteroaryloxy group and an alkylthio group, a combination of an arylthio group and an alkylthio group, a combination of a heteroarylthio group and an alkylthio group, a combination of an aryloxycarbonyl group and an alkylthio group, a combination of a heteroaryloxycarbonyl group and an alkylthio group, a combination of an arylthiocarbonyl group and an alkylthio group, a combination of a heteroarylthiocarbonyl group and an alkylthio group, a combination of an arylcarbonyloxy group and an alkylthio group, a combination of a heteroarylcarbonyloxy group and an alkylthio group, a combination of an arylcarbonylthio group and an alkylthio group, a combination of a heteroarylcarbonylthio group and an alkylthio group, a combination of an acyl group and an alkylthio group, and a combination of a heteroaryl group and an alkylthio group.

The number of substituent groups which can be included in $R^1$ is not particularly limited even in any one of the above-described aspects, and is appropriately set, preferably, in accordance with the kind of substituent groups. For example, in a case where the substituent group selected from the substituent-group group Z is a halogen atom, the number of substituent groups is preferably 1 to 37 (2 to 37 in a case of a composite substituent group), more preferably 1 to 19 (2 to 19 in a case of the composite substituent group), still more preferably 1 to 13 (2 to 13 in a case of the composite substituent group), and still more preferably 1 to 7 (2 to 7 in a case of the composite substituent group). In a case where the substituent group selected from the substituent-group group Z is an alkoxy group or an alkylthio group, the number of substituent groups selected from the substituent-group group Z is preferably 1 to 8 (2 to 8 in a case of a composite substituent group), and more preferably 1 to 4 (2 to 4 in a case of the composite substituent group). In a case where the substituent group selected from the substituent-group group Z is a substituent group other than a halogen atom, an alkoxy group, and an alkylthio group, the number of substituent groups is preferably 1 to 6 (2 to 6 in a case of a composite substituent group), and more preferably 1 to 3 (2 or 3 in a case of the composite substituent group).

In Formula (1), $R^{1a}$ represents a hydrogen atom, an alkyl group, a cycloalkyl group, an alkenyl group, an alkynyl group, an aryl group, a heteroaryl group, or an aliphatic heterocyclic group.

The alkyl group, the cycloalkyl group, the alkenyl group, the alkynyl group, the aryl group, the heteroaryl group, and the aliphatic heterocyclic group, which can be employed as $R^{1a}$, are the same as the alkyl group, the cycloalkyl group, the alkenyl group, the alkynyl group, the aryl group, the heteroaryl group, and the aliphatic heterocyclic group which can be employed as $R^1$, and preferred ranges thereof are the same as in the respective groups. Two pieces of $R^{1a}$, which are linked to a N atom and are adjacent to each other, may be linked to each other to form a ring. In this case, the ring that is formed may include a hetero atom as a ring-constituting atom.

$R^{1a}$ is preferably a hydrogen atom or an alkyl group, and more preferably a hydrogen atom.

Specific examples of the organic cation (A1) that can be included in the perovskite compound used in the invention will be described below, but the invention is not limited to the examples.

In the following specific examples, Me represents a metal.

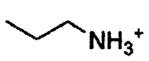
a1

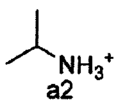
a2

$C_8H_{17}$-$NH_3^+$
a3

$C_{18}H_{37}$-$NH_3^+$
a4

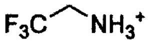
a5

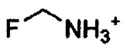
a6

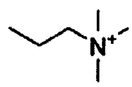
a7

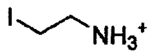
a8

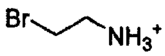
a9

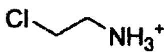
a10

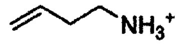
a11

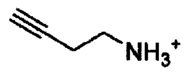
a12

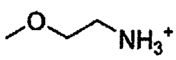
a13

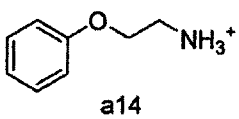
a14

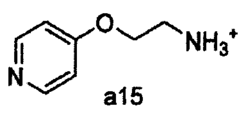
a15

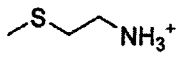
a16

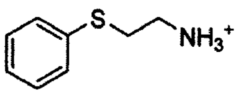
a17

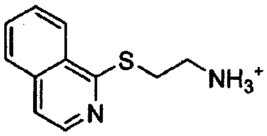
a18

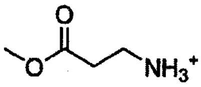
a19

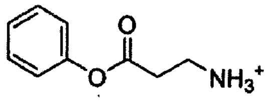
a20

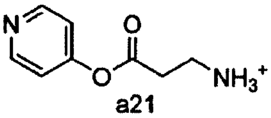
a21

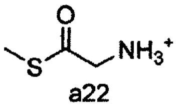
a22

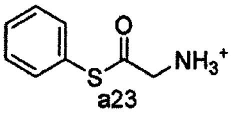
a23

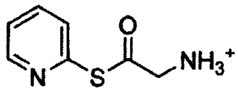
a24

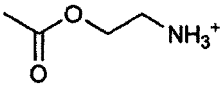
a26

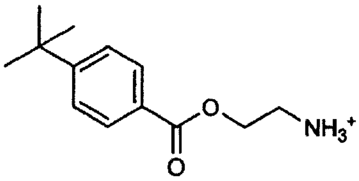
a27

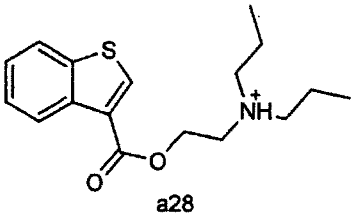
a28

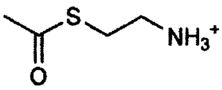
a29

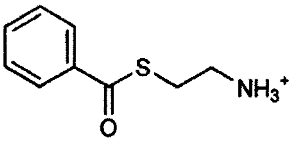
a30

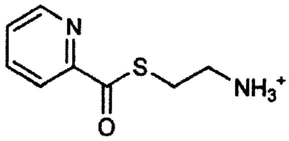
a31

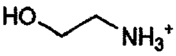
a32

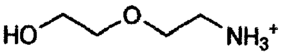
a33

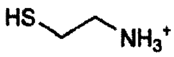
a34

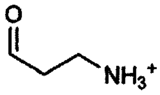
a35

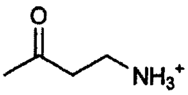
a36

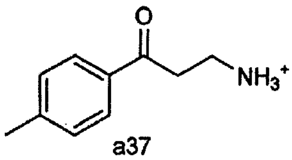
a37

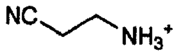
a38

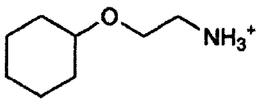
a39

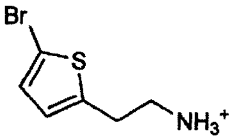
a40

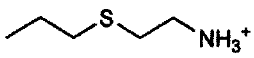
a41

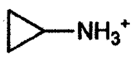
a42

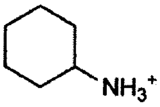
a43

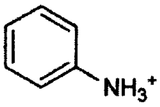
a44

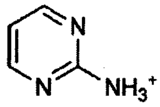
a45

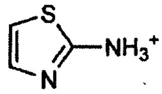
a46

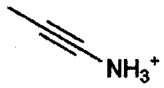
a47

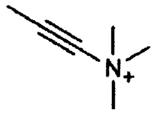
a48

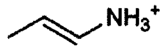
a49

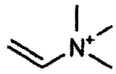
a50

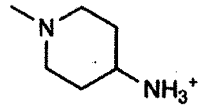
a51

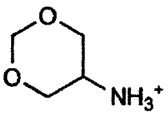
a52

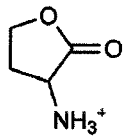
a53

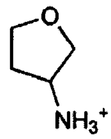
a54

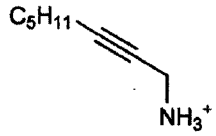
a55

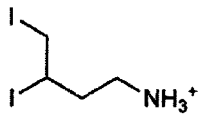
a56

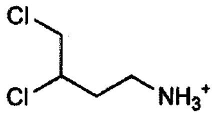
a57

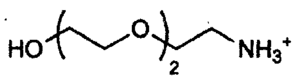
a58

The organic cation (A2) is represented by the following Formula (2).

Formula (2) $R^2\text{-}N(R^{2a})_3^+$

In Formula (2), $R^{2a}$ represents a hydrogen atom, an alkyl group, a cycloalkyl group, an alkenyl group, an alkynyl group, an aryl group, a heteroaryl group, or an aliphatic heterocyclic group.

The alkyl group, the cycloalkyl group, the alkenyl group, the alkynyl group, the aryl group, the heteroaryl group, and the aliphatic heterocyclic group, which can be employed as $R^{2a}$, are the same as the alkyl group, the cycloalkyl group, the alkenyl group, the alkynyl group, the aryl group, the heteroaryl group, and the aliphatic heterocyclic group which can be employed as $R^1$, and preferred ranges thereof are the same as in the respective groups. Two pieces of $R^{2a}$, which are linked to a N atom and are adjacent to each other, may be linked to each other to form a ring. In this case, the ring that is formed may include a hetero atom as a ring-constituting atom.

$R^{2a}$ is preferably a hydrogen atom or an alkyl group, and more preferably a hydrogen atom.

In Formula (2), $R^2$ represents a methyl group, an ethyl group, or a group represented by the following Formula (2a).

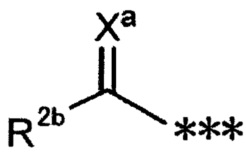

Formula (2a)

In a group represented by Formula (2a), $X^{2a}$ represents $NR^{2c}$, an oxygen atom, or a sulfur atom, and $NR^{2c}$ is preferable as $X^a$. Here, $R^{2c}$ represents a hydrogen atom or a substituent group. Although not particularly limited, as a substituent group that can be employed as $R^{2c}$, an alkyl group, a cycloalkyl group, an alkenyl group, an alkynyl group, an aryl group, a heteroaryl group, or an aliphatic heterocyclic group is preferable, and a hydrogen atom is more preferably.

$R^{2b}$ represents a hydrogen atom or a substituent group, and the hydrogen atom is preferable as $R^{2b}$. Although not particularly limited, examples of a substituent group that can be employed as $R^{2b}$ include an amino group, an alkyl group, a cycloalkyl group, an alkenyl group, an alkynyl group, an aryl group, a heteroaryl group, and an aliphatic heterocyclic group.

The alkyl group, the cycloalkyl group, the alkenyl group, the alkynyl group, the aryl group, the heteroaryl group, and the aliphatic heterocyclic group, which can be individually employed as $R^{2b}$ and $R^{2c}$ are the same as the alkyl group, the cycloalkyl group, the alkenyl group, the alkynyl group, the aryl group, the heteroaryl group, and the aliphatic heterocyclic group which can be employed as $R^1$ in Formula (1) and preferred ranges thereof are the same as in the respective

groups.

Examples of the group represented by Formula (2a) include a (thio)acyl group, a (thio)carbamoyl group, an imidoyl group, and an amidino group.

The (thio)acyl group includes an acyl group and a thioacyl group. The acyl group is preferably an acyl group having a total of 1 to 7 carbon atoms, and examples thereof include formyl, acetyl ($CH_3C(=O)-$), propionyl, hexanoyl, and the like. The thioacyl group is preferably a thioacyl group having a total of 1 to 7 carbon atoms, and examples thereof include thioformyl, thioacetyl ($CH_3C(=S)-$), thiopropionyl, and the like.

The (thio)carbamoyl group includes a carbamoyl group ($H_2NC(=O)-$) and a thiocarbamoyl group ($H_2NC(=S)-$).

The imidoyl group is a group represented by $R^{2b}-C(=NR^{2c})-$, and it is preferable that $R^{2b}$ and $R^{2c}$ are respectively a hydrogen atom and an alkyl group. More preferably, the alkyl group is the same as the alkyl group as $R^1$. Examples thereof include formimidoyl ($HC(=NH)-$), acetoimidoyl ($CH_3C(=NH)-$), propionimidoyl ($CH_3CH_2C(=NH)-$), and the like. Among these, formimidoyl is preferable.

The amidino group as the group represented by Formula (2) has a structure ($-C(=NH)NH_2$) in which $R^{2b}$ of the imidoyl group is an amino group and $R^{2c}$ is a hydrogen atom.

*** represents a bonding portion with a N atom in Formula (2).

In the invention, in a case where the entirety of three pieces of $R^{2a}$ are hydrogen atoms, the organic cation (A2) becomes an organic ammonium cation through bonding of $R^2$ and $NH_3$ in Formula (2). In a case where the organic ammonium cation can employ a resonance structure, the organic cation (A2) further includes a cation having the resonance structure in addition to the organic ammonium cation. For example, in a case where $X^a$ is NH ($R^{2c}$ is a hydrogen atom) in a group represented by Formula (2a), the organic cation also includes an organic amidinium cation that is one of a resonance structure of the organic ammonium cation in addition to the organic ammonium cation. Examples of the organic amidinium cation include a cation represented by the following Formula ($A^{am}$). In this specification, the cation represented by the following Formula ($A^{am}$) may be noted as "$R^{2b}C(=NH)-NH_3^{+}$" for convenience.

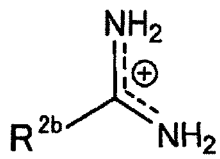

Formula ($A^{am}$)

In the perovskite compound that is used in the invention, it is preferable that a molar ratio of the amount of the organic cation (A2) contained to the amount of the organic cation (A1) contained satisfies a relationship defined in the following Expression (i), and more preferably a relationship defined in the following Expression (ii).

In the following Expressions (i) and (ii), [organic cation represented by Formula (2)] represents the amount of the organic cation (A2) contained in terms of a molar amount, and [organic cation represented by Formula (1)] represents the amount of the organic cation (A1) contained in terms of a molar amount.

Expression (i)

4≤[organic cation represented by Formula (2)]/[organic cation represented by Formula (1)]≤499

Expression (ii)

19≤[organic cation represented by Formula (2)]/[organic cation represented by Formula (1)]≤199

Since the molar ratio of the amount of the organic cation (A2) contained to the amount of the organic cation (A1) contained satisfies the relationships defined in Expression (i) or (ii), it is possible to further reduce the moisture resistance variation in the photoelectric conversion element.

In the perovskite compound that is used in the invention, a cation of the metal atom M is a cation of a metal atom other than elements of Group 1 in the periodic table, and it is preferable that the metal atom is a metal atom capable of employing a perovskite-type crystal structure. Examples of the metal atom M include metal atoms of calcium (Ca), strontium (Sr), cadmium (Cd), copper (Cu), nickel (Ni), manganese (Mn), iron (Fe), cobalt (Co), palladium (Pd), germanium

(Ge), tin (Sn), lead (Pb), ytterbium (Yb), europium (Eu), indium (In), titanium (Ti), bismuth (Bi), and thallium (Tl). Among these, the Pb atom, the Cu atom, the Ge atom, or the Sn atom is more preferable.

The cation of the metal atom M may be one kind of cation, or two or more kinds of cations. In a case of two or more kinds of cations, a ratio (ratio between the amounts of the cations contained) is not particularly limited.

In the perovskite compound that is used in the invention, the anion X represents an anion of an anionic atom or atomic group X. Preferred examples of the anion include anions of halogen atoms, and anions of individual atomic groups of $NC^-$, $NCS^-$, $NCO^-$, $OH^-$, $NO_3^-$, $CH_3COO^-$, and $HCOO^-$. Among these, the anions of halogen atoms are more preferable. Examples of the halogen atoms include a fluorine atom, a chlorine atom, a bromine atom, an iodine atom, and the like.

The anion X may be an anion of one kind of anionic atom or atomic group, or anions of two or more kinds of anionic atoms or atomic groups. In a case where the anion X is an anion of one kind of anionic atom or atomic group, an anion of an iodine atom is preferable. On the other hand, in a case where the anion X includes anions of two or more kinds of anionic atoms or atomic groups, anions of two kinds of halogen atoms, particularly, an anion of a chlorine atom and an anion of an iodine atom are preferable. A ratio between two or more kinds of anions (ratio between the amounts of anions contained) is not particularly limited.

The perovskite compound that is used in the invention has a perovskite-type crystal structure including the above-described constituent ions, and a perovskite compound represented, for example, by the following Formula (I) is preferable.

Formula (I) $A_aM_mX_x$

In Formula (I), A represents a cationic organic group. M represents a metal atom. X represents an anionic atom or atomic group.

a represents 1 or 2, m represents 1, and a, m, and x satisfy a relationship of a+2m=x.

In Formula (I), the cationic organic group forms the organic cation A of the perovskite-type crystal structure. The cationic organic group includes one or more kinds of cationic organic groups which generate the organic cation (A1) and one or more kinds of cationic organic groups which generate the organic cation (A2). The cationic organic groups, a molar ratio thereof, and the like are the same as those described in the organic cation A, and preferred examples thereof are the same as described above.

The metal atom M is a metal atom that forms the metal cation M having a perovskite-type crystal structure. Accordingly, the metal atom M is not particularly limited as long as the metal atom M is an atom that becomes the metal cation M and constitutes the perovskite-type crystal structure. The metal atom M is the same as the metal atom that is described in the metal cation M, and preferred examples thereof are the same as described above.

The anionic atom or atomic group X forms the anion X of the perovskite-type crystal structure. Accordingly, the anionic atom or atomic group X is not particularly limited as long as the anionic atom or atomic group X is an atom or atomic group that becomes the anion X and can constitute the perovskite-type crystal structure. The anionic atom or atomic group X is the same as the anionic atom or atomic group which is described in the above-described anion X, and preferred examples thereof are the same as described above.

The perovskite compound represented by Formula (1) is a perovskite compound represented by the following Formula (1-1) in a case where a is 1, or a perovskite compound represented by the following Formula (1-2) in a case where a is 2.

Formula (1-1): $AMX_3$

Formula (I-2): $A_2MX_4$

In Formula (I-1) and Formula (1-2), A represents a cationic organic group. A is the same as the cationic organic group A in Formula (I), and preferred examples thereof are the same as described above. M represents a metal atom. M is the same as the metal atom M in Formula (I), and preferred examples thereof are the same as described above. X represents an anionic atom or atomic group. X is the same as the anionic atom or atomic group in Formula (I), and preferred examples thereof are the same as described above.

The perovskite compound may further include an element of Group 1 in the periodic table or a cationic organic group other than cationic organic groups which become the organic cations (A1) and (A2) as an element or an organic group that becomes a cation.

Cations of elements of Group 1 in the periodic table are not particularly limited, and examples thereof include cations ($Li^+$, $Na^+$, $K^+$, and $Cs^+$) of respective elements of lithium (Li), sodium (Na), potassium (K), and cesium (Cs). Among these, a cation ($Cs^+$) of cesium is preferable.

The cation other than the organic cation (A1) and the organic cation (A2) is not particularly limited as long as

the cations are cations having the above-described property.

In the perovskite compound that is used in the invention, it is preferable that a ratio of a total molar amount of the organic cation (A1), the organic cation (A2), and the cation of the metal atom M to a total molar amount of cations which constitute the perovskite-type crystal structure is 90 to 100 mol%, and more preferably 95 to 100 mol%.

In addition, in the perovskite compound that is used in the invention, it is preferable that a ratio of a total molar amount of anions of halogen atoms to a total molar amount of anions which constitute the perovskite-type crystal structure is 90 to 100 mol%, more preferably 95 to 100 mol%, and still more preferably 98 to 100 mol%.

For example, the perovskite compound can be synthesized from a compound represented by the following Formula (II) and a compound represented by the following Formula (III).

Formula (II): AX

Formula (III): $MX_2$

In Formula (II), A represents a cationic organic group. A is the same as the cationic organic group A in Formula (I), and preferred examples thereof are the same as described above. In Formula (II), X represents an anionic atom or atomic group. X is the same as the anionic atom or atomic group X in Formula (I), and preferred examples thereof are the same as described above.

In Formula (III), M represents a metal atom. M is the same as the metal atom M in Formula (I), and preferred examples thereof are the same as described above. In Formula (III), X represents an anionic atom or atomic group. X is the same as the anionic atom or atomic group X in Formula (I), and preferred examples thereof are the same as described above.

Examples of a method of synthesizing the perovskite compound include a method described in Science, 2012, vol. 338, p. 643 to 647, Angew. Chem. Int. Ed. 2014, 53, p. 11232 to 11235, and Science, 2014, vol. 345, p. 295. Another example thereof also includes a method described in Akihiro Kojima, Kenjiro Teshima, Yasuo Shirai, and Tsutomu Miyasaka, "Organometal Halide Perovskites as Visible-Light Sensitizers for Photovoltaic Cells", J. Am. Chem. Soc., 2009, 131(17), p. 6050 to 6051.

The amount of the light absorbing agent used may be set to an amount capable of covering at least a part of the surface of the first electrode 1, and preferably an amount capable of covering the entirety of the surface.

The amount of the perovskite compound contained in the photosensitive layer 13 is typically 1% to 100% by mass.

<Hole Transport Layer 3>

As in the photoelectric conversion elements 10A to 10D, in a preferred aspect of the photoelectric conversion element of the invention, the hole transport layer 3 is provided between the first electrode 1 and the second electrode 2. In the aspect, it is preferable that the hole transport layer 3 is in contact with (laminated on) the photosensitive layer 13C. The hole transport layer 3 is preferably provided between the photosensitive layer 13 of the first electrode 1 and the second electrode 2.

The hole transport layer 3 includes a function of supplementing electrons to an oxidized substance of the light absorbing agent, and is preferably a solid-shaped layer (solid hole transport layer).

A hole transporting material that forms the hole transport layer 3 may be a liquid material or a solid material, and there is no particular limitation thereto. Examples of the hole transporting material include inorganic materials such as CuI and CuNCS, organic hole transporting materials described in Paragraphs 0209 to 0212 of JP2001-291534A, and the like. Preferred examples of the organic hole transporting material include conductive polymers such as polythiophene, polyaniline, polypyrrole, and polysilane, spiro compounds in which two rings share a central atom such as C or Si having a tetrahedral structure, aromatic amine compounds such as triarylamine, triphenylene compounds, nitrogen-containing heterocyclic compounds, and liquid-crystalline cyano compounds.

As the hole transporting material, an organic hole transporting material which can be applied in a solution state and then has a solid shape is preferable, and specific examples thereof include 2,2',7,7'-tetrakis-(N,N-di-p-methoxyphenylamino)-9,9'-spirobifluorene (also referred to as "spiro-MeOTAD"), poly(3-hexylthiophene-2,5-diyl), 4-(diethylamino)benzaldehyde diphenylhydrazone, polyethylene dioxythiophene (PEDOT), and the like.

Although not particularly limited, the film thickness of the hole transport layer 3 is preferably 50 μm or less, more preferably 1 nm to 10 μm, still more preferably 5 nm to 5 μm, and still more preferably 10 nm to 1 μm. Furthermore, the film thickness of the hole transport layer 3 corresponds to an average distance between the second electrode 2 and a surface of the photosensitive layer 13, and can be measured by observing a cross-section of the photoelectric conversion element by using a scanning electron microscope (SEM) and the like.

<Electron Transport Layer 4>

As in the photoelectric conversion element 10E, in a preferred aspect of the photoelectric conversion element of the invention, the electron transport layer 4 is provided between the first electrode 1 and the second electrode 2. In this aspect, it is preferable that the electron transport layer 4 is in contact with (laminated on) the photosensitive layer 13C.

The electron transport layer 4 is the same as the electron transport layer 15 except that an electron transporting destination is the second electrode, and a formation position is different.

<Second Electrode 2>

The second electrode 2 functions as a positive electrode or a negative electrode in a photoelectric conversion element. The second electrode 2 is not particularly limited as long as the second electrode 2 has conductivity. Typically, the second electrode 2 can be configured to have the same configuration as that of the conductive support 11. In a case where sufficient strength is maintained, the support 11a is not necessary.

As a structure of the second electrode 2, a structure having a high current-collection effect is preferable. At least one of the conductive support 11 or the second electrode 2 needs to be substantially transparent so that light reaches the photosensitive layer 13. In the photoelectric conversion element of the invention, as one of preferred aspects, the conductive support 11 is transparent and solar light and the like are incident from the support 11a side. In this aspect, it is more preferable that the second electrode 2 has a light-reflecting property.

Examples of a material used to form the second electrode 2 include metals such as platinum (Pt), gold (Au), nickel (Ni), copper (Cu), silver (Ag), indium (In), ruthenium (Ru), palladium (Pd), rhodium (Rh), iridium (Ir), osmium (Os), and aluminum (Al), the above-described conductive metal oxides, carbon materials, conductive polymers, and the like. The carbon materials may be conductive materials formed through bonding of carbon atoms, and examples thereof include fullerene, a carbon nanotube, graphite, graphene, and the like.

As the second electrode 2, a thin film (including a thin film obtained through vapor deposition) of a metal or a conductive metal oxide, or a glass substrate or a plastic substrate which has the thin film is preferable. As the glass substrate or the plastic substrate, glass including a gold or platinum thin film or glass on which platinum is vapor-deposited is preferable.

The film thickness of the second electrode 2 is not particularly limited, and is preferably 0.01 to 100 $\mu$m, more preferably 0.01 to 10 $\mu$m, and still more preferably 0.01 to 1 $\mu$m.

<Other Configurations>

In the invention, a spacer or a separator can also be used instead of the blocking layer 14 or in combination with the blocking layer 14 and the like so as to prevent the first electrode 1 and the second electrode 2 from coming into contact with each other.

In addition, a hole blocking layer may be provided between the second electrode 2 and the hole transport layer 3.

«Solar Cell»

The solar cell of the invention is constituted by using the photoelectric conversion element of the invention. For example, as illustrated in Fig. 1 to Fig. 6, the photoelectric conversion element 10 constituted by providing the external circuit 6 can be used as the solar cell. As the external circuit 6 that is connected to the first electrode 1 (the conductive support 11) and the second electrode 2, a known circuit can be used without particular limitation.

For example, the invention is applicable to respective solar cells described in Science, 2012, vol. 338, p. 643 to 647, Angew. Chem. Int. Ed. 2014, 53, p. 11232 to 11235, Science, 2014, vol. 345, p. 295, J. Am. Chem. Soc., 2009, 131(17), p. 6050 to 6051, and Science, 338, p. 643(2012).

It is preferable that a lateral surface of the solar cell of the invention is sealed with a polymer, an adhesive, and the like so as to prevent deterioration, evaporation, and the like in constituent substances.

«Method of Manufacturing Photoelectric Conversion Element and Solar Cell»

The photoelectric conversion element and the solar cell of the invention can be manufactured in accordance with a known method, for example, a method described in Science, 2012, vol. 338, p. 643 to 647, Angew. Chem. Int. Ed. 2014, 53, p. 11232 to 11235, Science, 2014, vol. 345, p. 295, J. Am. Chem. Soc., 2009, 131(17), p. 6050-6051, and Science, 338, p. 643(2012) except for formation of the photosensitive layer.

Hereinafter, the method of manufacturing the photoelectric conversion element and the solar cell of the invention will be described in brief.

In the manufacturing method of the invention, first, at least one of the blocking layer 14, the porous layer 12, the electron transport layer 15, or the hole transport layer 16 is formed on a surface of the conductive support 11 according to the purpose.

For example, the blocking layer 14 can be formed by a method in which a dispersion, which contains the insulating substance or a precursor compound thereof, and the like, is applied to the surface of the conductive support 11, and the dispersion is baked, a spray pyrolysis method, and the like.

A material that forms the porous layer 12 is preferably used as fine particles, and more preferably a dispersion that contains the fine particles.

A method of forming the porous layer 12 is not particularly limited, and examples thereof include a wet-type method, a dry-type method, and other methods (for example, a method described in Chemical Review, Vol. 110, p. 6595 (published on 2010)). In these methods, it is preferable that the dispersion (paste) is applied to the surface of the conductive support 11 or the surface of the blocking layer 14 and then the dispersion is baked at a temperature 100°C to 800°C for ten minutes to ten hours, for example, in the air. According to this, it is possible to bring the fine particles into close contact with each other.

In a case where baking is performed a plurality of times, a temperature in baking except final baking (a baking temperature except for a final baking temperature) is preferably set to be lower than the temperature in the final baking (the final baking temperature). For example, in a case where titanium oxide paste is used, the baking temperature except for the final baking temperature can be set in a range of 50°C to 300°C. In addition, the final baking temperature can be set in a range of 100°C to 600°C to be higher than the baking temperature except for the final baking temperature. In a case where a glass support is used as the support 11a, the baking temperature is preferably 60°C to 500°C.

The amount of a porous material applied to form the porous layer 12 is appropriately set in correspondence with the film thickness of the porous layer 12, the number of times of coating, and the like, and there is no particular limitation thereto. For example, the amount of the porous material applied per surface area 1 $m^2$ of the conductive support 11 is preferably 0.5 to 500 g, and more preferably 5 to 100 g.

In a case where the electron transport layer 15 or the hole transport layer 16 is provided, the layer can be formed in the same manner as in the hole transport layer 3 or the electron transport layer 4 to be described below.

Subsequently, the photosensitive layer 13 is provided.

In a case of forming the photosensitive layer 13, a compound capable of synthesizing a perovskite compound is used.

Examples of the compound capable of synthesizing a perovskite compound include a compound AX represented by Formula (II), and a compound $MX_2$ represented by Formula (III). The compounds may be used alone, or as a composition (including types such as solution, suspension, and paste).

In the invention, the photosensitive layer 13 can be formed by using a light absorbing agent composition that contains the entirety of the above-described compounds. In addition, the photosensitive layer 13 can be formed by using a composition that contains a compound represented by the following formula (1b) (the composition is a composition of the invention. For convenience, the composition may be referred to as an ammonium salt composition for discrimination from a light absorbing agent composition), a compound represented by the following formula (2b), and a compound $MX_2$.

In the compound AX represented by Formula (II) and the compound $MX_2$ represented by Formula (III), A, M, and X are the same as A, M, and X in Formula (I).

In the invention, it is preferable to use a compound $R^1\text{-}N(R^{1a})_3X$ and a compound $R^2\text{-}N(R^{2a})_3X$ as the compound AX. Here, $R^1$, $R^2$, $R^{1a}$, and $R^{2a}$ are the same as $R^1$, $R^2$, $R^{1a}$, and $R^{2a}$ in Formulae (1) and (2), and preferred examples thereof are the same as described above. In the compounds, X is preferably a halogen atom. In this case, the compounds are compounds represented by the following Formulae (1b) and (2b), and preferred examples thereof are the same as preferred examples of the following compounds.

Formula (1b) $R^1\text{-}N(R^{1a})_3Hal$

Formula (2b) $R^2\text{-}N(R^{2a})_3Hal$

In Formulae (1) and (2), $R^1$, $R^2$, $R^{1a}$, and $R^{2a}$ are the same as $R^1$, $R^2$, $R^{1a}$, and $R^{2a}$ in Formulae (1) and (2), and preferred examples thereof are the same as described above. Hal represents a halogen atom, and preferably an iodine atom, a chlorine atom, or a bromine atom.

The light absorbing agent composition contains the compound AX and the compound $MX_2$.

The light absorbing agent composition can be prepared by mixing the compound AX and the compound $MX_2$ in a predetermined molar ratio and by heating the resultant mixture. The formation liquid is typically a solution (light absorbing agent solution), but may be a suspension. Heating conditions are not particularly limited. For example, a heating temperature is preferably 30°C to 200°C, more preferably 60°C to 150°C, and still more preferably 70°C to 150°C. Heating time is preferably 0.5 to 100 hours, and more preferably 1 to 3 hours. As a solvent or a dispersion

medium, the following solvent or dispersion medium can be used.

As long as the ammonium salt composition includes one kind of the compound represented by Formula (1b) and one kind of the compound represented by Formula (2b), the ammonium salt composition may include two or more kinds of the compounds.

The ammonium salt composition may contain the compound $MX_2$, preferably, a halogenated metal (in a case where X is a halogen atom). In this case, the ammonium salt composition becomes a preferred aspect of the light absorbing agent composition.

Examples of the halogenated metal include halides (that is, compounds represented by $M(Hal)_2$, Hal represents a halogen atom), which are included in the perovskite compound used in the invention, of the metal atom M. The halogenated metal is preferably at least one kind selected from a halide of Pb and a halide of Sn, more preferably at least one kind selected from an iodide of Pb, a chlorinated matter of Pb, an iodide of Sn, and a chlorinated matter of Sn, and still more preferably at least one kind selected from $PbI_2$ and $SnI_2$.

In a case where the ammonium salt composition does not contain the compound $MX_2$, a metal salt composition, which contains the compound $MX_2$ and preferably a halogenated metal, is used. The metal salt composition is preferably a solution, and is prepared through heating as necessary. Heating conditions are not particularly limited. For example, a heating temperature is preferably 30°C to 200°C, and more preferably 50°C to 120°C. Heating time is preferably 5 minutes to 48 hours, and more preferably 0.5 to 24 hours.

The light absorbing agent composition, the ammonium salt composition, and the metal salt composition may have a solid shape (a powder, a granule, and the like), but a solution is preferable. In a case where the compositions are solutions, an organic solvent is preferable as a medium that is used. The organic solvent will be described later.

The compositions may further include another composition in addition to the above-described compounds. Examples of the other composition include halides of elements of Group 1 in the periodic table, and the like.

The compositions may be appropriately used as a supply source of the compound $MX_2$ and the compound AX in a case of forming the photosensitive layer in the photoelectric conversion element of the invention. In a case where the compositions are powders, granules, and the like, each of the compositions can be used after preparing a solution having an appropriate concentration by dissolving the composition in a solvent, and performing filtration, purification, and the like as necessary. In addition, in a case where the compositions are solution, the compositions can be used as is or after performing concentration, dilution, filtration, purification, and the like.

The entirety of the compositions can be appropriately used in a case of forming the photosensitive layer in manufacturing of the photoelectric conversion element of the invention.

In the light absorbing agent composition and the ammonium salt composition, it is preferable that the amount al of a compound represented by $R^1\text{-}N(R^{1a})_3X$ and the amount a2 of a compound represented by $R^2\text{-}N(R^{2a})_3X$ satisfy a molar ratio relationship defined in the following Expression (r1-1), more preferably a molar ratio relationship defined in the following Expression (r1-2), and still more preferably a molar ratio relationship defined in the following Expression (r1-3).

$$\text{Expression (r1-1)} \qquad 0.001 \leq a2/a1 \leq 999$$

$$\text{Expression (r1-2)} \qquad 4 \leq a2/a1 \leq 499$$

$$\text{Expression (r1-3)} \qquad 19 \leq a2/a1 \leq 199$$

In addition, in the light absorbing agent composition, the ammonium salt composition, and the metal salt composition, the amount a1 of the compound represented by $R^1\text{-}N(R^{1a})_3X$, the amount a2 of the compound represented by $R^2\text{-}N(R^{2a})_3X$, and the amount c of the compound $MX_2$ are appropriately set in a range capable of forming the perovskite compound. In a case of forming the perovskite compound, typically, the sum of the amount al and the amount a2 is set to be excessive with respect to the amount c. A specific used amount may not be specified in some cases, for example, in accordance with the following method in which the ammonium salt composition and the metal salt composition are individually applied. In the invention, in a case where the amount c can be specified, it is preferable that the amounts a1, a2, and c satisfy a molar ratio relationship defined in the following Expression (r2-1), and more preferably a molar ratio relationship defined in the following Expression (r2-2).

$$\text{Expression (r2-1)} \qquad 1 \leq (a1+a2)/c \leq 10$$

$$\text{Expression (r2-2)} \qquad 1 \leq (a1+a2)/c \leq 5$$

In the light absorbing agent composition, the ammonium composition, and the metal salt composition, a molar ratio a1 :a2:c between the amount al of the compound represented by $R^1$-N($R^{1a}$)$_3$X, the amount a2 of the compound represented by $R^2$-N($R^{2a}$)$_3$X, and the amount c of the compound $MX_2$ can be set by appropriately combining respective Expressions (r1-1) to (r1-3), and respective expressions of Expressions (r2-1) and (r2-2).

A concentration of a solid content of each of the light absorbing agent composition, the ammonium salt composition, and the metal salt composition is not particularly limited. For example, the concentration of the solid content of the light absorbing agent composition is preferably 0.1% to 99% by mass, and more preferably 1% to 70% by mass. The concentration of the solid content of the ammonium salt composition is preferably 0.05% to 90% by mass, and more preferably 0.1% to 55% by mass. The concentration of the solid content of the metal salt composition is preferably 0.05% to 95% by mass, and more preferably 0.1% to 80% by mass.

Examples of a method of providing the photosensitive layer 13 include a wet-type method and a dry-type method, and there is no particular limitation thereto. In the invention, the wet-type method is preferable, and for example, a method of bringing the light absorbing agent composition or the ammonium salt composition into contact with a surface of a layer (in the photoelectric conversion element 10, any one of the porous layer 12, the blocking layer 14, the electron transport layer 15, and the hole transport layer 16) on which the photosensitive layer 13 is formed is preferable.

In the method of bringing the light absorbing agent composition into contact with the surface, specifically, it is preferable that the light absorbing agent composition is applied to the surface or the surface is immersed in the light absorbing agent composition. A contact temperature is preferably 5°C to 100°C, and immersion time is preferably 5 seconds to 24 hours and more preferably 20 seconds to 1 hour. In a case of drying the light absorbing agent composition that is applied, with regard to the drying, drying with heat is preferable, and drying is performed by heating the applied light absorbing agent composition typically at 20°C to 300°C, and preferably at 50°C to 170°C.

In addition, the photosensitive layer can also be formed in conformity to a method of synthesizing the perovskite compound.

Preferred examples of the method of bringing the ammonium salt composition into the surface include a method in which the ammonium salt composition and the metal salt composition are individually applied to the surface (including an immersion method), and are dried as necessary. In this method, an arbitrary composition may be applied to the surface in advance, but it is preferable that the metal salt composition is applied to the surface in advance. Application conditions and drying conditions in this method are the same as in the method of bringing the light absorbing agent composition into contact with the surface. In the method of bringing the ammonium salt composition into contact with the surface, the ammonium salt composition or the metal salt composition may be vapor-deposited instead of application of the ammonium salt composition and the metal salt composition.

As another method, a dry-type method such as a vacuum vapor deposition, in which a mixture from which a solvent of the light absorbing agent composition is removed is used, may be exemplified. For example, a method in which the compound AX and the compound $MX_2$ are simultaneously or sequentially vapor-deposited may be exemplified.

The perovskite compound is formed (deposited, adsorbed, or the like) on the surface of the porous layer 12, the blocking layer 14, the electron transport layer 15, or the hole transport layer 16 as the photosensitive layer through the above-described method and the like.

The hole transport layer 3 or the electron transport layer 4 is preferably formed on the photosensitive layer 13 that is prepared as described above.

The hole transport layer 3 can be formed by applying and drying a hole transporting material solution that contains a hole transporting material. In the hole transporting material solution, the concentration of the hole transporting material is preferably 0.1 to 1.0 M (mol/L) when considering that application properties are excellent, and in a case where the porous layer 12 is provided, the hole transporting material is capable of easily intruding into pores in the porous layer 12.

The electron transport layer 4 can be formed by applying and drying an electron transporting material solution that contains an electron transporting material.

After the hole transport layer 3 or the electron transport layer 4 is formed, the second electrode 2 is formed, thereby manufacturing the photoelectric conversion element.

The film thicknesses of the respective layers can be adjusted by appropriately changing the concentrations of respective dispersion liquids or solutions and the number of times of application. For example, in a case where the photosensitive layers 13B and 13C having a large film thickness are provided, the light absorbing agent composition, the ammonium composition, or the metal salt composition may be applied and dried a plurality of times.

The respective dispersion liquids and solutions described above may respectively contain additives such as a dispersion auxiliary agent and a surfactant as necessary.

Examples of the solvent or dispersion medium that is used in the method of manufacturing the photoelectric

conversion element and the solar cell include a solvent described in JP2001-291534A, but the solvent or dispersion medium is not particularly limited thereto. In the invention, an organic solvent is preferable, and an alcohol solvent, an amide solvent, a nitrile solvent, a hydrocarbon solvent, a lactone solvent, a halogen solvent, a sulfide solvent, and a mixed solvent of two or more kinds thereof are preferable. As the mixed solvent, a mixed solvent of the alcohol solvent and a solvent selected from the amide solvent, the nitrile solvent, and the hydrocarbon solvent is preferable. Specifically, methanol, ethanol, isopropanol, γ-butyrolactone, n-propylsulfide, chlorobenzene, acetonitrile, N,N-dimethylformamide (DMF), dimethylacetamide, and a mixed solvent thereof are preferable.

A method of applying the solutions or dispersants which form the respective layers is not particularly limited, and it is possible to use a known application method such as spin coating, extrusion die coating, blade coating, bar coating, screen printing, stencil printing, roll coating, curtain coating, spray coating, dip coating, an inkjet printing method, and an immersion method. Among these, the spin coating, the screen printing, the immersion method, and the like are preferable.

The photoelectric conversion element of the invention may be subjected to an efficiency stabilizing treatment such as annealing, light soaking, and being left as is in an oxygen atmosphere as necessary.

The photoelectric conversion element prepared as described above can be used as a solar cell after connecting the external circuit 6 to the first electrode 1 (transparent electrode 11b) and the second electrode 2.

Hereinafter, the invention will be described in more detail on the basis of examples, but the invention is not limited to the following examples.

## Examples

### (Example 1)

The photoelectric conversion element 10A illustrated in Fig. 1 was prepared in the following procedure. A case where the film thickness of the photosensitive layer 13 is large corresponds to the photoelectric conversion element 10B illustrated in Fig. 2.

[Manufacturing of Photoelectric Conversion Element (Sample No. 101)]

<Preparation of Conductive Support 11 >

A fluorine-doped $SnO_2$ conductive film (the transparent electrode 11b, film thickness: 300 nm) was formed on a glass substrate (the support 11a, thickness: 2.2 mm), thereby preparing the conductive support 11.

<Preparation of Solution for Blocking Layer>

15% by mass isopropanol solution of titanium diisopropoxide bis(acetylacetonate) (manufactured by Sigma-Aldrich Co. LLC) was diluted with 1-butanol, thereby preparing 0.02 M solution for a blocking layer.

<Formation of Blocking Layer 14>

The blocking layer 14 (film thickness: 100 nm) that consists of titanium oxide was formed on the $SnO_2$ conductive film of the conductive support 11 by using the prepared 0.02 M solution for the blocking layer at 450°C in accordance with a spray pyrolysis method.

<Preparation of Titanium Oxide Paste>

Ethyl cellulose, lauric acid, and terpineol were added to an ethanol dispersion liquid of titanium oxide (anatase, an average particle size: 20 nm), thereby preparing titanium oxide paste.

<Formation of Porous Layer 12>

The prepared titanium oxide paste was applied onto the blocking layer 14 with a screen printing method, and was baked. Application and baking of the titanium oxide paste were respectively performed twice. With regard to a baking temperature, first baking was performed at 130°C, and second baking was performed at 500°C for 1 hour. A baked body of the titanium oxide, which was obtained, was immersed in 40 mM $TiCl_4$ aqueous solution, and was heated at 60°C for 1 hour, and heating was continuously performed at 500°C for 30 minutes, thereby forming the porous layer 12 (film thickness: 250 nm) formed from $TiO_2$.

<Formation of Photosensitive Layer 13A>

A 40% methanol solution (27.86 mL) of methyl amine, and an aqueous solution of 57% by mass of hydrogen iodide (hydroiodic acid: 30 mL) were stirred in a flask at 0°C for 2 hours, and were concentrated to obtain coarse $CH_3NH_3I$. The obtained coarse $CH_3NH_3I$ was dissolved in ethanol and was recrystallized with diethylether. A precipitated crystal was filtered and collected, and was dried under reduced pressure at 60°C for 24 hours, thereby obtaining purified $CH_3NH_3I$ (compound that is represented by Formula (2b) and generates the organic cation (A2). The compound may be referred to as a compound (2b)).

A 10% ethanol solution (100 g) of the following compound (b1), and an aqueous solution (38 g) of 57% by mass of hydrogen iodide were stirred in a flask at 0°C for 2 hours, and the resultant mixture was concentrated to remove a solvent, and was dried under reduced pressured at 60°C for 24 hours, thereby obtaining the following compound c1 (compound that is represented by Formula (1b) and generates the organic cation (A1). The compound may be referred to as a compound (1b)).

Then, purified $CH_3NH_3I$, the compound c1, and $PbI_2$ (halogenated metal) were stirred and mixed in DMF at 60°C for 12 hours in a ratio in which a mixing molar ratio (a2/a1) was set to 99, a mixing molar ratio ((a1+a2)/c) was set to 1.01, and a mixing molar ratio a1:a2:c was set to 99:1:99.5. Then, the resultant mixture was filtered with a polytetrafluoroethylene (PTFE) syringe filter, thereby preparing, thereby preparing a 40% by mass light absorbing agent solution A (ammonium salt composition that contains a halogenated metal).

The prepared light absorbing agent solution A was applied onto the porous layer 12 with a spin coating method (for 60 seconds at 2000 rpm) (application temperature: 60°C), and the applied light absorbing agent solution A was dried by using a hot plate at 100°C for 90 minutes, thereby forming the photosensitive layer 13A (film thickness: 300 nm (including the film thickness of 250 nm of the porous layer 12)) including a perovskite compound.

In this manner, the first electrode 1 was prepared.

<Preparation of Hole Transporting Material Solution>

Spiro-MeOTAD (180 mg) as the hole transporting material was dissolved in chlorobenzene (1 mL). 37.5 μL of an acetonitrile solution obtained by dissolving lithium-bis (trifluoromethanesulfonyl) imide (170 mg) in acetonitrile (1 mL) and t-butyl pyridine (TBP, 17.5 μL) were additionally mixed to the chlorobenzene solution, thereby preparing a hole transporting material solution.

<Formation of Hole Transport Layer 3>

Subsequently, the hole transporting material solution was applied onto the photosensitive layer 13 of the first electrode 1 by using a spin coating method, and the hole transporting material solution, which was applied, was dried to form the hole transport layer 3 (film thickness: 0.1 μm).

<Preparation of Second Electrode 2>

Gold (film thickness: 0.1 μm) was vapor-deposited on the hole transport layer 3 by a vapor deposition method, thereby preparing the second electrode 2.

In this manner, the photoelectric conversion element 10 of Sample No. 101 was manufactured.

Respective film thicknesses were measured through observation with a SEM according to the above-described method.

[Manufacturing of Photoelectric Conversion Elements (Sample Nos. 102 to 108)]

Photoelectric conversion elements 10 of Sample Nos. 102 to 108 were manufactured in the same manner as in the manufacturing of the photoelectric conversion element of Sample No. 101 except that the mixing molar ratios (a2/al) and ((al+a2)/c) between purified $CH_3NH_3I$ and the compound c1, and the mixing molar ratio (al:a2:c) between the compound (2b), the compound (1b), and $PbI_2$ were changed to values illustrated in Table 2 in comparison to the manufacturing of the photoelectric conversion element of Sample No. 101.

[Manufacturing of Photoelectric Conversion Elements (Sample Nos. 109 to 133)]

Photoelectric conversion elements 10 of Sample Nos. 109 to 133 were manufactured in the same manner as in the manufacturing of the photoelectric conversion element of Sample No. 101 except that a compound (1b) illustrated in Table 2 was used instead of the compound c1, and the mixing molar ratios (a2/a1), ((a1+a2)/c), and the mixing molar

ratio (a1:a2:c) were set to values illustrated in Table 2 in comparison to the manufacturing of the photoelectric conversion element of Sample No. 101.

Here, as the compounds b1 to b54, a commercially available product was used, or a compound obtained from appropriate synthesis was used.

In the manufacturing of the photoelectric conversion element of Sample Nos. 109 to 113, 115 to 130, and 133, respective compounds c2 to c6, c8 to c11, c13, c14, c16, c32, c40, c43, c44, c46, and c54, which were synthesized from compounds b2 to b6, b8 to b11, b13, b14, b16, b32, b40, b43, b44, b46, and b54, was used as the compound (1b) in the same manner as in the manufacturing of the photoelectric conversion element of Sample No. 101.

In addition, the compound c7, which was used in the manufacturing of the photoelectric conversion element of Sample No. 114, was synthesized in conformity to a method described in Journal of the American Chemical Society, 1986, vol. 108, #23, p. 7141 to 7147. The compound c48, which was used in the manufacturing of the photoelectric conversion element of Sample No. 131, was synthesized in conformity to Journal of Organic Chemistry, 1971, vol. 36, p. 3856 to 3861. The compound c50, which was used in the manufacturing of the photoelectric conversion element of Sample No. 132, was obtained from Tokyo Chemical Industry Co., Ltd.

[Manufacturing of Photoelectric Conversion Element (Sample No. 134)]

A photoelectric conversion element 10 of Sample No. 134 was manufactured in the same manner as in the manufacturing of the photoelectric conversion element of Sample No. 101 except that purified $HC(=NH)NH_3I$, which was synthesized by using formamidine instead of methylamine, was used as the compound (2b) in comparison to the manufacturing of the photoelectric conversion element of Sample No. 101.

[Manufacturing of Photoelectric Conversion Element (Sample No. 135)]

A photoelectric conversion element 10 of Sample No. 135 was manufactured in the same manner as in the manufacturing of the photoelectric conversion element of Sample No. 101 except that the photosensitive layer 13A was formed by the following method in comparison to the manufacturing of the photoelectric conversion element of Sample No. 101.

$PbI_2$ was stirred in DMF at 70°C for 12 hours, thereby obtaining a 40% by mass $PbI_2$ solution (metal salt composition). The solution was applied onto the porous layer 12 by a spin coating method (for 60 seconds at 2000 rpm) (application temperature: 60°C). The applied $PbI_2$ solution was dried by using a hot plate at 100°C for 60 minutes, thereby forming a $PbI_2$ film.

Subsequently, a 1% by mass isopropanol solution (composition of the invention), which was obtained by mixing purified $CH_3NH_3I$ as the compound (2b) and the compound c1 as the compound (1b) in a molar ratio of 99:1, was prepared. The $PbI_2$ film was immersed in the solution (immersion temperature: 20°C) for 30 seconds, and was dried at 100°C for 60 minutes by using a hot plate, thereby forming the photosensitive layer 13A (film thickness: 300 nm (including the film thickness of 250 nm of the porous layer 12)).

Although not specifically determined, the sum of the amount a1 of the compound c1 contained and the amount a2 of purified $CH_3NH_3I$ contained with respect to the amount c of $PbI_2$ contained was excessive in formation of the photosensitive layer 13A.

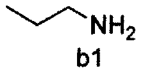
b1
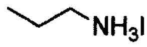
c1
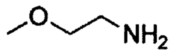
b13
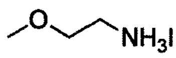
c13

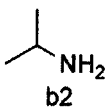
b2
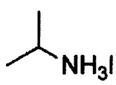
c2
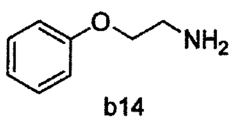
b14
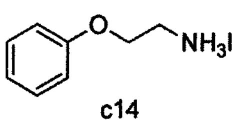
c14

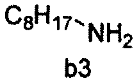
b3
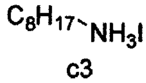
c3
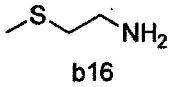
b16
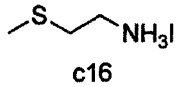
c16

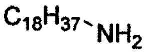
b4
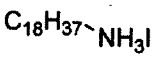
c4
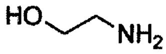
b32
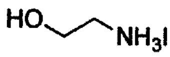
c32

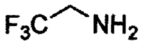
b5
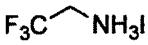
c5
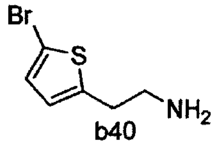
b40
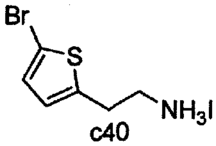
c40

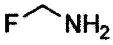
b6
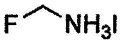
c6
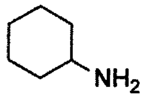
b43
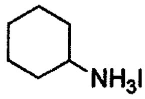
c43

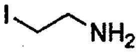
b8
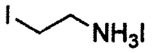
c8
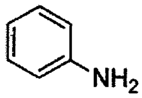
b44
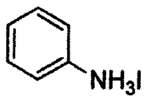
c44

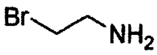
b9
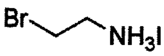
c9
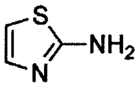
b46
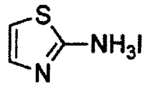
c46

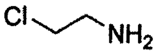
b10
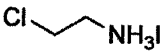
c10
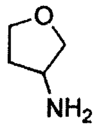
b54
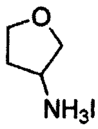
c54

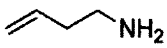
b11
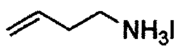
c11

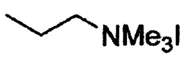
c7
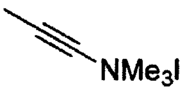
c48
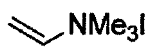
c50

[Manufacturing of Photoelectric Conversion Element (Sample No. c11)]

A photoelectric conversion element 10 of Sample No. c11 was manufactured in the same manner as in the manufacturing of the photoelectric conversion element of Sample No. 101 except that the compound c1 was not used in the light absorbing agent solution, the mixing molar ratio between purified $CH_3NH_3I$ (compound (2b)) and $PbI_2$ was set to 100:100, and the mixing molar ratio ((a1+a2)/c) was set to a value illustrated in Table 2 in comparison to the manufacturing of the photoelectric conversion element of Sample No. 101.

[Manufacturing of Photoelectric Conversion Element (Sample No. c12)]

A photoelectric conversion element 10 of Sample No. c12 was manufactured in the same manner as in the manufacturing of the photoelectric conversion element of Sample No. 101 except that purified $CH_3NH_3I$ (compound (2b)) was not used in the light absorbing agent solution, the mixing molar ratio between the compounds c1 and $PbI_2$ was set to 100:50, and the mixing molar ratio ((a1+a2)/c) was set to a value illustrated in Table 2 in comparison to the manufacturing of the photoelectric conversion element of Sample No. 101.

[Manufacturing of Photoelectric Conversion Elements (Sample Nos. c13 and c14)]

Photoelectric conversion elements 10 of Sample Nos. c13 and c14 were manufactured in the same manner as in the manufacturing of the photoelectric conversion element of Sample No. 101 except that the following compound (zc1) or (zc2) illustrated in Table 2 was used instead of the compound c1 as a compound that generates an organic cation (A3), and a mixing molar ratio (a2/zc) between purified $CH_3NH_3I$ (compound (2b)) and the compound (zc1) or (zc2) and a mixing molar ratio ((zc+a2)/c) (described in a column of "((al+a2)/c)" in Table 2) were set to values illustrated in Table 2 in comparison to the manufacturing of the photoelectric conversion element of Sample No. 101.

Here, the compound (zc1) or (zc2) was synthesized from the following compound (zb1) or (zb2) in the same manner as in the <Formation of Photosensitive Layer 13A> in the photoelectric conversion element of Sample No. 101.

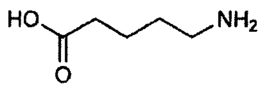

Zb1

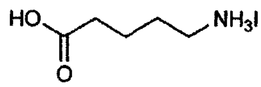

Zc1

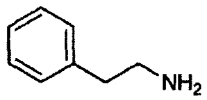

Zb2

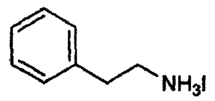

Zc2

[Manufacturing of Photoelectric Conversion Element (Sample No. c15)]

A photoelectric conversion element 10 of Sample No. c15 was manufactured in the same manner as in the manufacturing of the photoelectric conversion element of Sample No. c11 except that a mixture of purified $CH_3NH_3I$ and purified $HC(=NH)NH_3I$ in a molar ratio of 99:1 was used as the compound (1b) instead of purified $CH_3NH_3I$ (compound (2b)) in comparison to the manufacturing of the photoelectric conversion element of Sample No. c11.

In the respective photoelectric conversion elements of the invention which were manufactured as described above, the molar ratio ([A2]/[A1]) between the organic cation (A1) and the organic cation (A2) which form the perovskite-type crystal structure can be substituted with the mixing molar ratio (a2/a1) of the light absorbing agent solution A, the ammonium salt composition, and the like. In the respective photoelectric conversion elements of Sample Nos. 101 to 135, the molar ratio between the organic cation (A1) and the organic cation (A2) in the photosensitive layer was approximately the same as the mixing molar ratio between the compound (1b) and the compound (2b). As analysis means at this time, gas chromatography-mass spectrometry (GC-MS) was used. In measurement of GC-MS, a sample may be subjected to a base treatment for detection as amine, or may be subjected to derivatization for detection as necessary. In a case where analysis is difficult with the means, GC-MS may be substituted with X-ray diffraction (XRD) or liquid chromatography mass analysis (LC-MS)

In addition, in the respective photoelectric conversion element of Sample Nos. 101 to 134, a molar ratio (([A1]+[A2])/[$PbI_2$]) was approximately the same as the mixing molar ratio ((a1+a2)/c) in the light absorbing agent solution A. In addition, the molar ratio [A2]:[A1]:[$PbI_2$] can be measured in the same manner by using ion chromatography (ICP) in addition to the method as necessary, and [A2]:[A1]:[$PbI_2$] was approximately the same as the mixing molar ratio a1 :a2:c.

In the respective photoelectric conversion elements (Sample Nos. 101 to 135) of the invention, a ratio of a total molar amount of the organic cation (A1), the organic cation (A2), and the metal cation to a total molar amount of cations which constitute the perovskite-type crystal structure was 100 mol% in each case. In addition, a ratio of a molar amount of anions of halogen atoms to a total molar amount of anions which constitute the perovskite-type crystal structure was 100 mol% in each case.

[Evaluation of Moisture Resistance Variation Under High Humidity]

<Measurement of Initial Photoelectric Conversion Efficiency>

Initial photoelectric conversion efficiency was evaluated as follows.

Seven specimens were manufactured for each of the photoelectric conversion elements of respective sample numbers. A battery characteristic test was performed with respect to each of the seven specimens to measure initial photoelectric conversion efficiency ($\eta^I$/%) (after manufacturing).

The battery characteristic test was performed through irradiation of pseudo-solar light of 1000 $W/m^2$ from a xenon lamp through an AM1.5 filter by using a solar simulator "WXS-85H" (manufactured by Wacom). The photoelectric

conversion efficiency (η/%) was obtained by measuring current-voltage characteristics by using an I-V tester.

At the initial photoelectric conversion efficiency ($\eta^I$/%) measured in each of the photoelectric conversion elements (Sample Nos. 101 to 135), a photoelectric conversion element or a solar cell can sufficiently function.

<Measurement of Photoelectric Conversion Efficiency Under High Humidity>

Photoelectric conversion efficiency (referred to as "photoelectric conversion efficiency under high humidity) when the photoelectric conversion element was left under high humidity under the following conditions was evaluated as follows.

Each of the seven specimens of photoelectric conversion elements for which the initial photoelectric conversion efficiency was measured was maintained in a constant-temperature and constant-humidity bath under a high-humidity environment of a temperature of 45°C and a humidity of 55%RH for 24 hours, and then a battery characteristic test was performed in the same manner as in <Measurement of Initial Photoelectric Conversion Efficiency> to measure photoelectric conversion efficiency ($\eta^R$/%) under high humidity.

In the photoelectric conversion efficiency ($\eta^R$/%) under high humidity which was measured in each of the photoelectric conversion elements (Sample Nos. 101 to 135), a deterioration amount with respect to initial photoelectric conversion efficiency ($\eta^I$) was small even after being maintained for 24 hours, and at the photoelectric conversion efficiency, a photoelectric conversion element or a solar cell could sufficiently function under the high-humidity.

<Evaluation of Moisture Resistance Variation Under High Humidity>

In the respective photoelectric conversion elements of the sample numbers, moisture resistance variation under high humidity of the photoelectric conversion elements was evaluated from the initial photoelectric conversion efficiency and the photoelectric conversion efficiency under high humidity as follows.

In each of the seven specimens of photoelectric conversion elements, a deterioration rate of the photoelectric conversion efficiency was calculated from the following expression, and an average value of the deterioration rates was set as an average deterioration rate. The average deterioration rate of the photoelectric conversion efficiency was set to 1.0 (reference), and a deterioration rate of the photoelectric conversion efficiency in each of the seven specimens with respect to the average deterioration rate was calculated as a relative value (relative deterioration rate). A difference (relative deterioration rate - 1.0) between the relative deterioration rate that was calculated and the average deterioration rate 1.0 as a reference was obtained in each of the specimens, and ranges, in which a difference having the greatest absolute value among obtained differences is included, were classified under the following evaluation criteria to evaluate the moisture resistance variation under high humidity. Results are illustrated in Table 2 as "moisture resistance variation"

$$\text{Deterioration rate (\%)} = 100 - \{100\times[(\text{photoelectric conversion efficiency under high humidity } \eta^R)/(\text{initial photoelectric conversion efficiency } \eta^I)]\}$$

- Evaluation Criteria of Moisture Resistance Variation Under High Humidity -

With regard to the maximum value among the differences of the relative deterioration rate, criteria are as follows.

A: Maximum value is in a range of ±0.18
B: Maximum value is beyond a range of ±0.18 and is in a range of ±0.21
C: Maximum value is beyond a range of ±0.21 and is in a range of ±0.24
D: Maximum value is beyond a range of ±0.24 and is in a range of ±0.28
E: Maximum value is beyond a range of ±0.28 and is in a range of ±0.32
F: Maximum value is beyond a range of ±0.32

[Table 2]

| Sample No. | Compound (1b) (organic cation (A1)) | Compound (2b) (organic cation (A2)) | Organic cation (A3) | [A2]/[A1] (a2/a1) | [A2]/[A3] (a2/zc) | [A2]:[A1]:[$PbI_2$] (a1:a2:c) | ((al+a2)/c) | Moisture resistance variation | Remarks |
|---|---|---|---|---|---|---|---|---|---|
| 101 | c1 | $CH_3NH_3I$ | - | 99 | - | 99:1:99.5 | 1.01 | B | Present Invention |
| 102 | c1 | $CH_3NH_3I$ | - | 0.01 | - | 1:99:50.5 | 1.98 | D | Present Invention |
| 103 | c1 | $CH_3NH_3I$ | - | 4 | - | 80:20:90 | 1.11 | C | Present Invention |
| 105 | c1 | $CH_3NH_3I$ | - | 9 | - | 90:10:95 | 1.05 | C | Present Invention |
| 104 | c1 | $CH_3NH_3I$ | - | 19 | - | 95:5:97.5 | 1.03 | B | Present Invention |
| 106 | c1 | $CH_3NH_3I$ | - | 199 | - | 99.5:0.5:99.8 | 1.00 | B | Present Invention |
| 107 | c1 | $CH_3NH_3I$ | - | 499 | - | 99.8:0.2:99.9 | 1.00 | C | Present Invention |
| 108 | c1 | $CH_3NH_3I$ | - | 999 | - | 99.9:0.1:100 | 1.00 | D | Present Invention |
| 109 | c2 | $CH_3NH_3I$ | - | 99 | - | 99:1:99.5 | 1.01 | B | Present Invention |
| 110 | c3 | $CH_3NH_3I$ | - | 99 | - | 99:1:99.5 | 1.01 | B | Present Invention |
| 111 | c4 | $CH_3NH_3I$ | - | 99 | - | 99:1:99.5 | 1.01 | B | Present Invention |
| 112 | c5 | $CH_3NH_3I$ | - | 99 | - | 99:1:99.5 | 1.01 | A | Present Invention |
| 113 | c6 | $CH_3NH_3I$ | - | 99 | - | 99:1:99.5 | 1.01 | A | Present Invention |
| 114 | c7 | $CH_3NH_3I$ | - | 99 | - | 99:1:99.5 | 1.01 | C | Present Invention |

(continued)

| Sample No. | Compound (1b) (organic cation (A1)) | Compound (2b) (organic cation (A2)) | Organic cation (A3) | [A2]/[A1] (a2/a1) | [A2]/[A3] (a2/zc) | [A2]:[A1]:[$PbI_2$] (a1:a2:c) | ((al+a2)/c) | Moisture resistance variation | Remarks |
|---|---|---|---|---|---|---|---|---|---|
| 115 | c8 | $CH_3NH_3I$ | - | 99 | - | 99:1:99.5 | 1.01 | A | Present Invention |
| 116 | c9 | $CH_3NH_3I$ | - | 99 | - | 99: 1:99.5 | 1.01 | A | Present Invention |
| 117 | c10 | $CH_3NH_3I$ | - | 99 | - | 99:1:99.5 | 1.01 | A | Present Invention |
| 118 | c11 | $CH_3NH_3I$ | - | 99 | - | 99:1:99.5 | 1.01 | B | Present Invention |
| 119 | c13 | $CH_3NH_3I$ | - | 99 | - | 99:1:995 | 1.01 | A | Present Invention |
| 120 | c14 | $CH_3NH_3I$ | - | 99 | - | 99:1:99.5 | 1.01 | B | Present Invention |
| 121 | c16 | $CH_3NH_3I$ | - | 9 | - | 90:10:95 | 1.05 | B | Present Invention |
| 122 | c16 | $CH_3NH_3I$ | - | 19 | - | 95:5:97.5 | 1.03 | A | Present Invention |
| 123 | cl6 | $CH_3NH_3I$ | - | 99 | - | 99:1:99.5 | 1.01 | A | Present Invention |
| 124 | c16 | $CH_3NH_3I$ | - | 199 | - | 99.5:0.5:99.8 | 1.00 | A | Present Invention |
| 125 | cl6 | $CH_3NH_3I$ | - | 499 | - | 99.8:0.2:99.9 | 1.00 | B | Present Invention |
| 126 | c32 | $CH_3NH_3I$ | - | 99 | - | 99:1:99.5 | 1.01 | B | Present Invention |
| 127 | c40 | $CH_3NH_3I$ | - | 99 | - | 99:1:99.5 | 1.01 | A | Present Invention |
| 128 | c43 | $CH_3NH_3I$ | - | 99 | - | 99:1:99.5 | 1.01 | B | Present Invention |

(continued)

| Sample No. | Compound (1b) (organic cation (A1)) | Compound (2b) (organic cation (A2)) | Organic cation (A3) | [A2]/[A1] (a2/a1) | [A2]/[A3] (a2/zc) | [A2]:[A1]:[$PbI_2$] (a1:a2:c) | ((al+a2)/c) | Moisture resistance variation | Remarks |
|---|---|---|---|---|---|---|---|---|---|
| 129 | c44 | $CH_3NH_3I$ | - | 99 | - | 99:1:99.5 | 1.01 | B | Present Invention |
| 130 | c46 | $CH_3NH_3I$ | - | 99 | - | 99:1:99.5 | 1.01 | B | Present Invention |
| 131 | c48 | $CH_3NH_3I$ | - | 99 | - | 99:1:99.5 | 1.01 | C | Present Invention |
| 132 | c50 | $CH_3NH_3I$ | - | 99 | - | 99:1:99.5 | 1.01 | C | Present Invention |
| 133 | c54 | $CH_3NH_3I$ | - | 99 | - | 99:1:99.5 | 1.01 | B | Present Invention |
| 134 | c1 | $HC(=NH)NH_3I$ | - | 99 | - | 99:1:99.5 | 1.01 | B | Present Invention |
| 135 | c1 | $CH_3NH_3I$ | - | 99 | - | 99:1:- | - | B | Present Invention |
| c11 | - | $CH_3NH_3I$ | - | - | - | 100:0:100 | 1.00 | F | Comparative Example |
| c12 | c1 | - | - | - | - | 0:100:50 | 2.00 | F | Comparative Example |
| c13 | - | $CH_3NH_3I$ | zc1 | - | 99 | 99:1:99.5 | 2.00 | E | Comparative Example |
| c14 | - | $CH_3NH_3I$ | zc2 | - | 99 | 99:1:99.5 | 1.01 | E | Comparative Example |
| c15 | - | $CH_3NH_3I$:$HC(=NH)NH_3I$=99:1 | - | - | - | 100:0:100 | 1.00 | E | Comparative Example |

In Table 2, [A2]/[A1] represents a molar ratio between the amount of the organic cation (A1) contained, and the amount of the organic cation (A2) contained. [A2]/[A3] represents a molar ratio between the amount of the organic cation (A2) contained, and the amount of the organic cation (A3) contained.

In addition, [A2]:[A1]:[$PbI_2$] represents a molar ratio between the amount of the organic cation (A1) contained, the amount of the organic cation (A2) contained, and the amount of $PbI_2$ contained.

From results in Table 2, it can be understood as follows.

Even in the photoelectric conversion elements using the perovskite compound as the light absorbing agent, in a case where the perovskite compound contains specific organic cations (A1) and (A2) as cations which constitute the perovskite-type crystal structure (Sample Nos. 101 to 135), even under a high-humidity environment of a temperature of 45°C and a humidity of 55%RH, the moisture resistance variation was small, and stable moisture resistance was exhibited. As described above, in a case of using two kinds of specific organic cations defined in the invention in combination as organic cations which form the perovskite-type crystal structure, it could be understood that it is possible to improve the moisture resistance variation under high humidity in the photoelectric conversion element.

In addition, in photoelectric conversion elements in which in the perovskite compound, the molar ratio ([A2]/[A1]) between the amount of the organic cation (A1) represented by Formula (1) and the amount of the organic cation (A2) represented by Formula (2) is in a range of 4 to 499 or in a range of 19 to 199, it was possible to further suppress the moisture resistance variation under high humidity in each case.

In a case where $R^1$ in Formula (1) includes at least one selected from the group consisting of an alkoxy group, an alkylthio group, and a halogen atom, even in a case where an aspect of including a substituent group pertains to any one of the aspects, a photoelectric conversion element, which includes the organic cation included in $R^1$, exhibited a moisture resistance variation that is further suppressed to be small even under high humidity.

Even in a case of forming the photosensitive layer by using the light absorbing agent composition, and even in a case of forming the photosensitive layer by using the ammonium salt composition, the moisture resistance variation under high humidity of the photoelectric conversion element was excellent.

In contrast, in a case where the perovskite compound does not contain two kinds of specific organic cations as cations which form the perovskite-type crystal structure (Sample Nos. c11 to c15), the moisture resistance variation under high humidity was great in each case. Particularly, even in photoelectric conversion elements of Sample No. c11 corresponding to Science, 2012, vol. 338, p. 643 to 647, Sample No. c13 corresponding to Angew. Chem. Int. Ed. 2014, 53, p. 11232 to 11235, and Sample No. c14 corresponding to Science, 2014, vol. 345, p. 295, it was difficult to sufficiently suppress the moisture resistance variation under high humidity.

The invention has been described in combination with embodiments thereof. However, it is not intended to limit the invention in any detailed part of the description unless particularly specified, and it should be understood that the invention is supposed to be widely interpreted within the spirit and the scope of the invention which are described in the appended claims.

Priority is claimed on Japanese Patent Application No. 2015-133860, filed July 2, 2015, the content of which is incorporated herein by reference.

Explanation of References

1A to 1F: first electrode
11: conductive support
11a: support
11b: transparent electrode
12: porous layer
13A to 13C: photosensitive layer
14: blocking layer
2: second electrode
3A, 3B, 16: hole transport layer
4, 15: electron transport layer
6: external circuit (lead)
10A to 10F: photoelectric conversion element
100A to 100F: system using solar cell
M: electric motor

## Claims

**1.** A photoelectric conversion element, comprising:

a first electrode that includes a photosensitive layer, which includes a light absorbing agent, on a conductive support; and
a second electrode that is opposite to the first electrode,
wherein the light absorbing agent includes a compound having a perovskite-type crystal structure that includes an organic cation, a cation of a metal atom, and an anion of an anionic atom or atomic group,
the organic cation includes an organic cation represented by the following Formula (1) and an organic cation represented by the following Formula (2),

Formula (1) $R^1\text{-}N(R^{1a})_3^+$

Formula (2) $R^2\text{-}N(R^{2a})_3^+$

in Formulae (1) and (2), $R^1$ represents an alkyl group, a cycloalkyl group, an alkenyl group, an alkynyl group, an aryl group, a heteroaryl group, or an aliphatic heterocyclic group, the alkyl group includes a substituent group, which is selected from the following substituent-group group Z, other than an alkyl group in a case where the number of carbons is 1 or 2,
$R^2$ represents a methyl group, an ethyl group, or a group represented by the following Formula (2a),
$R^{1a}$ and $R^{2a}$ each independently represent a hydrogen atom, an alkyl group, a cycloalkyl group, an alkenyl group, an alkynyl group, an aryl group, a heteroaryl group, or an aliphatic heterocyclic group,

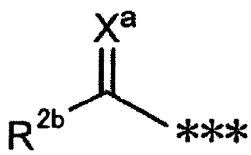

Formula (2a)

in Formula (2a), $X^a$ represents $NR^{2c}$, an oxygen atom, or a sulfur atom, $R^{2b}$ and $R^{2c}$ each independently represent a hydrogen atom or a substituent group, *** represents a bonding portion with a N atom in Formula (2), and
substituent-group group Z: an alkyl group, an alkenyl group, an alkynyl group, an alkoxy group, an aryloxy group, a heteroaryloxy group, an alkylthio group, an arylthio group, a heteroarylthio group, an alkoxycarbonyl group, an aryloxycarbonyl group, a heteroaryloxycarbonyl group, an alkylthiocarbonyl group, an arylthiocarbonyl group, a heteroarylthiocarbonyl group, an alkylcarbonyloxy group, an arylcarbonyloxy group, a heteroarylcarbonyloxy group, an alkylcarbonylthio group, an arylcarbonylthio group, a heteroarylcarbonylthio group, a hydroxy group, a mercapto group, an acyl group, a halogen atom, a cyano group, and a heteroaryl group.

**2.** The photoelectric conversion element according to claim 1,

wherein in $R^1$, the alkyl group includes a substituent group, which is selected from the substituent-group group Z, other than an alkyl group.

**3.** The photoelectric conversion element according to claim 1 or 2,

wherein $R^1$ includes at least one selected from the group consisting of an alkoxy group, an alkylthio group, and a halogen atom.

**4.** The photoelectric conversion element according to any one of claims 1 to 3,

wherein the amount of the organic cation that is contained and is represented by Formula (1), and the amount

of the organic cation that is contained and is represented by Formula (2) satisfy a molar ratio relationship defined by the following expression,

$$4 \leq [\text{organic cation represented by Formula (2)}]/[\text{organic cation represented by Formula (1)}] \leq 499.$$

**5.** The photoelectric conversion element according to any one of claims 1 to 4,

wherein the amount of the organic cation that is contained and is represented by Formula (1), and the amount of the organic cation that is contained and is represented by Formula (2) satisfy a molar ratio relationship defined by the following expression,

$$19 \leq [\text{organic cation represented by Formula (2)}]/[\text{organic cation represented by Formula (1)}] \leq 199.$$

**6.** A solar cell that uses the photoelectric conversion element according to any one of claims 1 to 5.

**7.** A composition, containing:

a compound that is represented by the following Formula (1b); and
a compound that is represented by the following Formula (2b),

Formula (1b) $R^1\text{-}N(R^{1a})_3Hal$

Formula (2b) $R^2\text{-}N(R^{2a})_3Hal$

in Formulae (1b) and (2b), $R^1$ represents an alkyl group, a cycloalkyl group, an alkenyl group, an alkynyl group, an aryl group, a heteroaryl group, or an aliphatic heterocyclic group, the alkyl group includes a substituent group, which is selected from the following substituent-group group Z, other than an alkyl group in a case where the number of carbons is 1 or 2,
$R^2$ represents a methyl group, an ethyl group, or a group represented by the following Formula (2a),
$R^{1a}$ and $R^{2a}$ each independently represent a hydrogen atom, an alkyl group, a cycloalkyl group, an alkenyl group, an alkynyl group, an aryl group, a heteroaryl group, or an aliphatic heterocyclic group,
Hal represents a halogen atom,

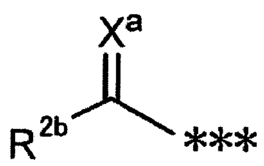

Formula (2a)

in Formula (2a), $X^a$ represents $NR^{2c}$, an oxygen atom, or a sulfur atom, $R^{2b}$ and $R^{2c}$ each independently represent a hydrogen atom or a substituent group, *** represents a bonding portion with a N atom in Formula (2b), and
substituent-group group Z: an alkyl group, an alkenyl group, an alkynyl group, an alkoxy group, an aryloxy group, a heteroaryloxy group, an alkylthio group, an arylthio group, a heteroarylthio group, an alkoxycarbonyl group, an aryloxycarbonyl group, a heteroaryloxycarbonyl group, an alkylthiocarbonyl group, an arylthiocarbonyl group, a heteroarylthiocarbonyl group, an alkylcarbonyloxy group, an arylcarbonyloxy group, a heteroarylcarbonyloxy group, an alkylcarbonylthio group, an arylcarbonylthio group, a heteroarylcarbonylthio group, a hydroxy group, a mercapto group, an acyl group, a halogen atom, a cyano group, and a heteroaryl

group.

**8.** The composition according to claim 7,

wherein in $R^1$, the alkyl group includes a substituent group, which is selected from the substituent-group group Z, other than an alkyl group.

**9.** The composition according to claim 7 or 8, further containing:
a halogenated metal.

**10.** The composition according to any one of claims 7 to 9,

wherein the composition is used to form the photosensitive layer of the photoelectric conversion element according to any one of claims 1 to 5.

FIG. 1

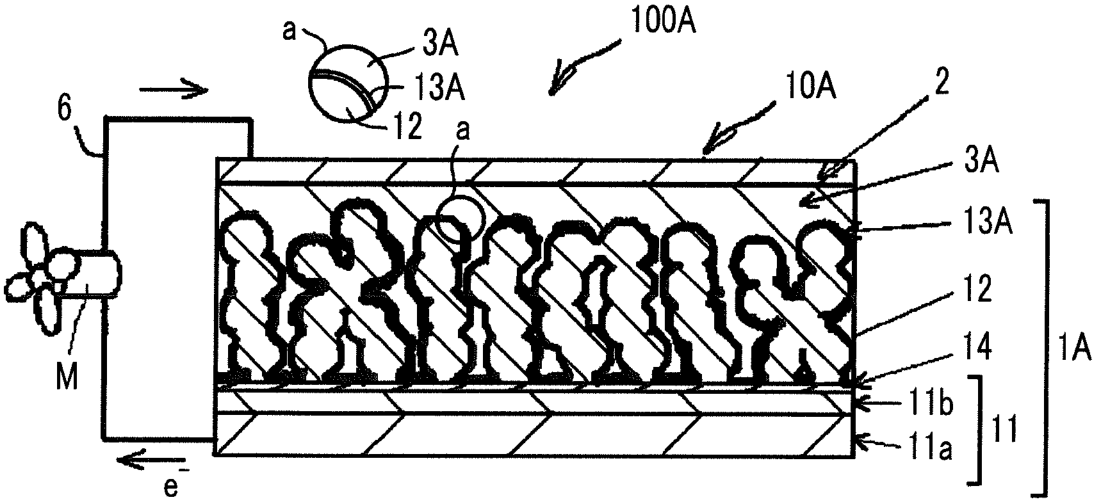
100A
a
3A
13A
12
a
6
10A
2
3A
13A
12
14
1A
11b
11
11a
M
e⁻

FIG. 2

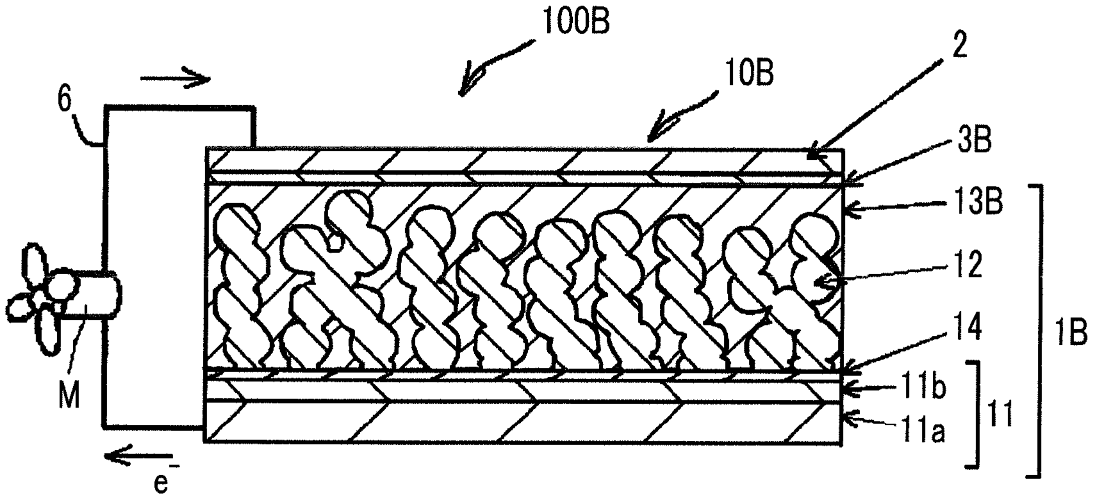
100B
6
10B
2
3B
13B
12
14
1B
11b
11
11a
M
e⁻

FIG. 3

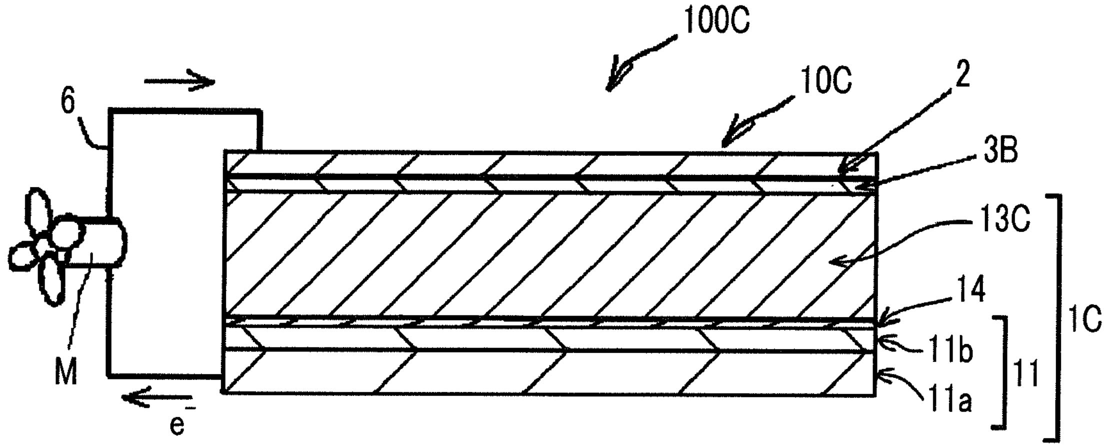
100C
10C
2
3B
13C
14
11b
11a
11
1C
6
M
e⁻

FIG. 4

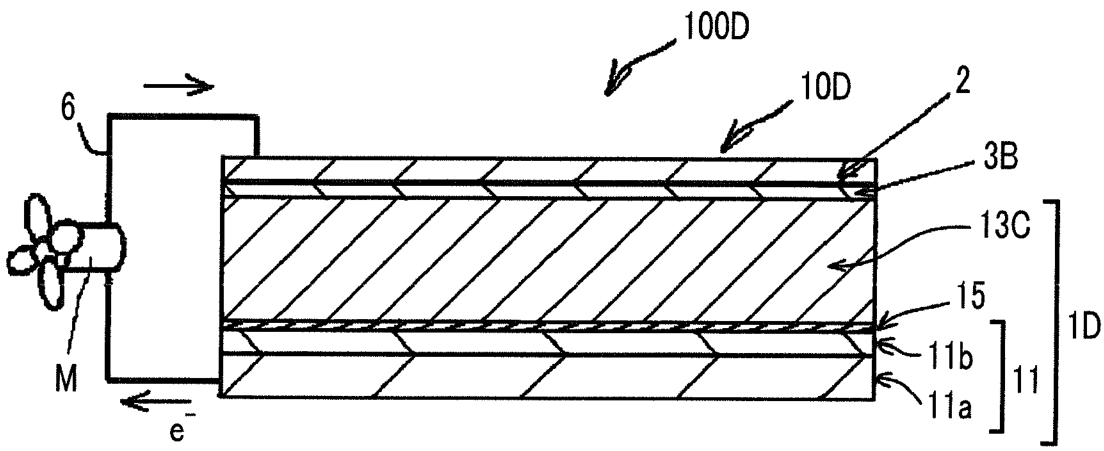
100D
10D
2
3B
13C
15
11b
11a
11
1D
6
M
e⁻

FIG. 5

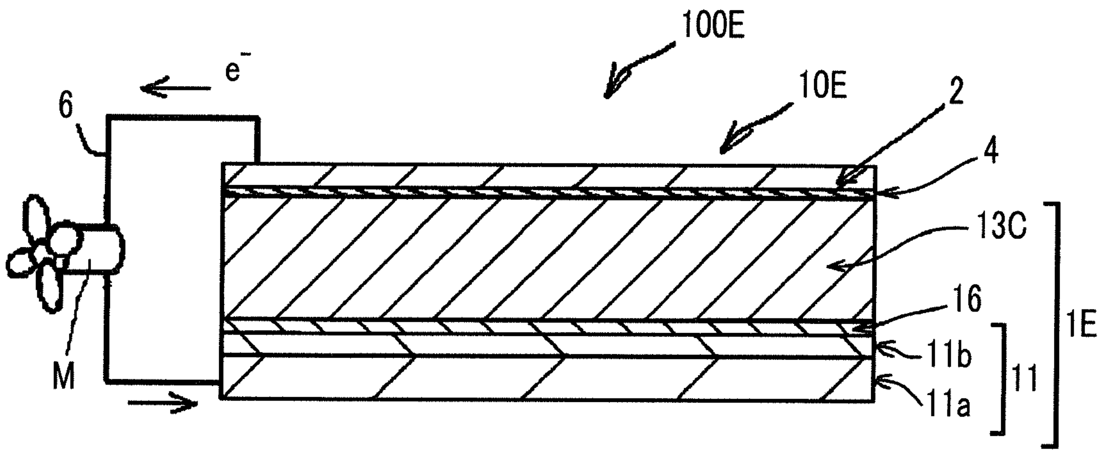
100E
e⁻
6
10E
2
4
13C
16
11b
11
1E
11a
M

FIG. 6

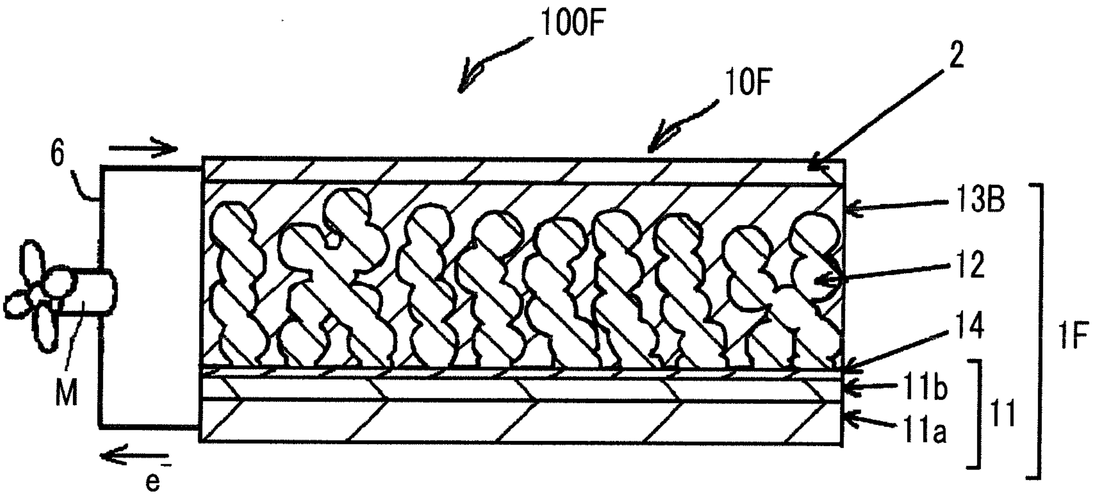
100F
10F
2
6
13B
12
14
1F
11b
11
11a
M
e⁻

## INTERNATIONAL SEARCH REPORT

International application No.
PCT/JP2016/069127

A. CLASSIFICATION OF SUBJECT MATTER
*H01L51/46*(2006.01)i, *H01L51/44*(2006.01)i

According to International Patent Classification (IPC) or to both national classification and IPC

B. FIELDS SEARCHED

Minimum documentation searched (classification system followed by classification symbols)
H01L51/46, H01L51/44

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>A | JP 2014-175472 A (Osaka Gas Co., Ltd.), 22 September 2014 (22.09.2014), claims; paragraphs [0011] to [0014] (Family: none) | 1,2,6-10<br>3-5 |

☐ Further documents are listed in the continuation of Box C.   ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 August 2016 (09.08.16) | 23 August 2016 (23.08.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2001291534 A
- US 4927721 A
- US 4684537 A
- US 5084365 A
- US 5350644 A
- US 5463057 A
- US 5525440 A
- JP 7249790 A
- JP H7249790 A
- JP 2004220974 A
- JP 2008135197 A
- JP 2005135902 A
- JP 2001160425 A
- JP 2003123859 A
- JP 2002260746 A
- JP 2015133860 A


### Non-patent literature cited in the description


- **ANGEW.** *Chem. Int. Ed.,* 2014, vol. 53, 11232-11235
- *Science,* 2014, vol. 345, 295
- *Science,* 2012, vol. 338, 643-647
- **J. AM.** *Chem. Soc.,* 2009, vol. 131 (17), 6050-6051
- *Science,* 2012, vol. 338, 643
- **ANGEW.** *Chem. Int. Ed.,* 2014, vol. 53, 11232-11235
- *J. Am. Chem. Soc.,* 2009, vol. 131 (17), 6050-6051
- *Angew. Chem. Int. Ed.,* 2014, vol. 53, 11232-11235
- **J. AM.** *Chem. Soc.,* 2009, vol. 131 (17), 6050-6051
- *Chemical Review,* 2010, vol. 110, 6595
- *Journal of the American Chemical Society,* 1986, vol. 108 (#23), 7141-7147
- *Journal of Organic Chemistry,* 1971, vol. 36, 3856-3861